(19) Europäisches Patentamt / European Patent Office / Office européen des brevets

(11) **EP 4 261 218 A1**

(12) **EUROPEAN PATENT APPLICATION**

(43) Date of publication:
**18.10.2023  Bulletin 2023/42**

(21) Application number: **22167691.9**

(22) Date of filing: **11.04.2022**

(51) International Patent Classification (IPC):
**C07K 1/00** $^{(2006.01)}$   **C07K 1/113** $^{(2006.01)}$
**G05B 13/00** $^{(2006.01)}$   **G06N 3/00** $^{(2023.01)}$
**G16B 15/20** $^{(2019.01)}$

(52) Cooperative Patent Classification (CPC):
**C07K 1/00; C07K 1/1136; G06N 3/00; G16B 15/20**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Bilfinger Life Science GmbH
5412 Puch/Salzburg (AT)**

(72) Inventors:
• **Pauk, Jan Niklas
  1160 Wien (AT)**
• **Berghammer, Gerald
  4984 Weilbach (AT)**
• **Igwe, Chika Linda
  1030 Wien (AT)**
• **Herwig, Christoph
  1130 Wien (AT)**

(74) Representative: **Ullrich & Naumann PartG mbB
Schneidmühlstrasse 21
69115 Heidelberg (DE)**

(54) **DIGITAL TWIN AND BIOREACTOR FOR PROTEIN REFOLDING PROCESSES**

(57)     The invention relates to a method for producing a protein comprising a refolding process, wherein the refolding process is carried out in a bioreactor and is monitored and/or controlled by means of a Digital Twin of the refolding process, wherein the Digital Twin comprises a state-observer algorithm in combination with a model of the refolding process based mathematical models of the reaction kinetics and known values of CPPs and process determinants selected from CQAs and KPIs of the refolding process. The invention relates to a method for optimizing the production process of a protein, comprising a refolding process, using a Digital Twin of the production process and a bioreactor for the refolding process of proteins, comprising a process vessel, a temperature control, a gassing unit for the addition of one or more of $N_2$, $O_2$ and process air, at least 5 addition groups, one or more sensors and an aseptic sampling port connected to an on-line or at-line measurement unit for one or more process determinants.

Inclusion Bodies    Solubilized Protein    Folding Intermediates    Native Protein    Aggregates

Fig. 1

**Description**

FIELD OF THE INVENTION

The application relates to a production method production of a protein, which includes a process of refolding proteins and the creation and use of a Digital Twin of the refolding process for optimizing and monitoring the process. The application further relates to a bioreactor for the refolding process.

BACKGROUND OF THE INVENTION

The biopharmaceutical industry has evidently huge potential, shown by an increase of the total annual revenue by 6250-fold from 1990 until 2018 (BioPharma Market: An Inside Look 2018) ranging over a wide variety of products (Pham et al. 2019). This increase results from the strong rise in FDA-approved recombinant drugs by 400 % from 2003 until 2018, as well as process optimization leading to enhanced product titers (Fifteen years of progress: biopharmaceutical industry survey results 2018). The expression of recombinant proteins amount to around 400 of around 650 worldwide approved protein drugs in 2016 (Sanchez-Garcia et al. 2016). Furthermore, these trends appear to be relatively stable and are predicted to continuously grow (BioPharma Market: An Inside Look 2018; Kesik-Brodacka 2018). Since many patents of the original innovative biopharmaceuticals are running out in the near future or have already been running out, the next strongly increasing market section are biosimilars - biopharmaceuticals similar to approved drugs on the market - or biobetters - biopharmaceuticals based on approved drugs with better functional or structural properties (Kesik-Brodacka 2018).

In the beginning of recombinant protein production in the early 1980s, *Escherichia coli* has been the major host for the production of biopharmaceuticals (Sanchez-Garcia et al. 2016). Although it has been overtaken by mammalian cell lines, bacterial hosts - especially *E. coli* - are still the second largest group of hosts amounting to 24-30 % overall production of biopharmaceuticals (Baeshen et al. 2014; Overton 2014).

*E. coli*'s pre-eminence is due to it being physiologically well characterized and recognized as host by regulatory authorities (Overton 2014). Furthermore, it yields high product titers with rapid growth and cost-effectiveness and is generally easy to use (Kesik-Brodacka 2018). Common drawbacks of bacterial hosts have been reviewed extensively (Overton 2014) including *E. coli*'s inability to perform posttranslational modifications such as glycosylation or phosphorylation (Kesik-Brodacka 2018). Furthermore, proteins containing disulfide bonds cannot fold correctly due to the reducing cytosolic environment of *E. coli,* hence, leading to aggregation and the formation of inclusion bodies (IBs) (Singh and Panda 2005). IB formation is further increased by stress due to overexpression and high inducer concentration (Singh and Panda 2005).

Originally, IBs were considered waste products, but recently this dogma has changed towards the realization of exploiting the IBs favorable properties (García-Fruitós 2010). Although solubilization and subsequent refolding is a tedious, costly, and often the most time-consuming downstream processing step, the IBs themselves contain the product in high yield and purity. In addition, encapsulation of the product in IBs enables easy separation from host cell proteins and protection from proteolytic activity (Pauk et al. 2021).

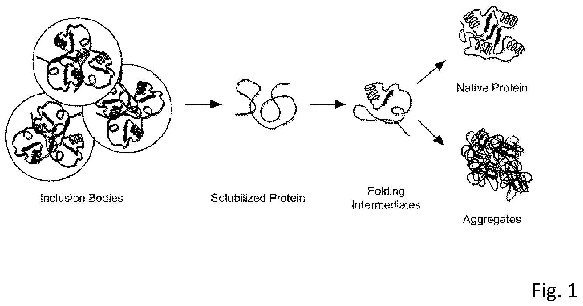

Protein refolding is the generalized term of transferring a misfolded or aggregated protein into its native state. This usually includes the solubilization - unfolding of the protein with denaturing agents such as urea or guanidine hydrochloride (GuHCl) and reducing agents such as dithiothreitol - followed by the refolding step where the solubilized protein is transferred into energetically favorable conditions (Singh and Panda 2005). After rapid reaction from the solubilized state to its folding intermediates, the protein dynamics compete between a folding reaction and an aggregation reaction (see Fig. 1) (Pan et al. 2015).

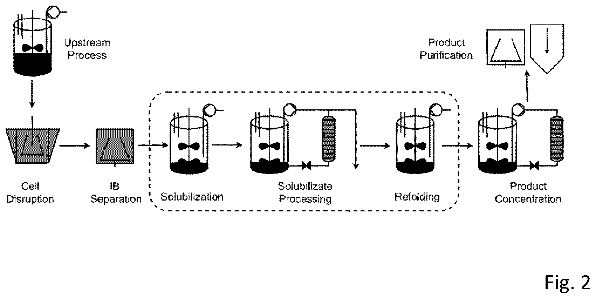

As undesired aggregate formation was found to be strongly dependent on the protein concentration, state-of-the art refolding is often done via dilution of the solubilized protein into huge amounts of refolding buffer. For this method, however, common drawbacks include low yields, challenging analytics, and necessary additional processing steps as it might be advantageous to concentrate or exchange buffers in between different unit operations (Fig. 2). To assure competitive product recovery and STYs, fed-batch processing is the method of choice to reduce volumes, and to assure refolding dynamics directed towards the native state of the target protein for elevated total protein concentrations (Eiberle and Jungbauer 2010). Further common refolding techniques include methods such as dialysis, diafiltration, on-column refolding or refolding by hydrostatic pressure (Pauk et al. 2021; Singhvi et al. 2020).

With the goal of setting common grounds for quality assurance, in 2004, the US Food and Drug Administration (FDA) launched its regulatory guidelines for pharmaceutical development named Process Analytical Technology (PAT). Thereby proposing principles enabling real-time process monitoring and in-process control based on in-depth process understanding (FDA 2004). Since then, a lot of effort has been targeted towards the implementation of efficient and sensitive analytical strategies suitable for real-time monitoring of bioprocesses (Rathore 2014; Gerzon et al. 2022).

However, application of those methods to protein refolding processes comes at quite a challenge due to various obstacles like extremely low protein concentrations, barely ranging up to 0.1 g L$^{-1}$ (Eiberle and Jungbauer 2010), and therefore being at the lower sensitivity limits and detection ranges of a broad spectrum of analytical methods. As the overall protein concentration in such processes usually include all states of the ongoing dynamics, like different states of soluble protein as well as insoluble fractions, analytical differentiation between the individual states is of even higher effort (Pathak et al. 2016). In addition, presence of denaturing agents at changing concentrations are critically interfering with protein specific wavelengths, reducing the applicability of common and already well-established spectroscopic methods (Guin et al. 2016). Still, various chromatographic and spectroscopic methods were shown to comprise sensitivity requirements that are suitable to fulfill the criteria necessary for application in refolding processes (Humer and Spadiut 2018). Therefore, several monitoring strategies have been implemented to observe state changes in refolding processes, mostly targeted at measuring an increase in the active state of the protein, often by chromatographic methods or simple activity assays (Humer et al. 2020; Chen et al. 2016; Schlegl et al. 2005; Bade et al. 2012). Furthermore, direct monitoring of structural changes in protein conformation has been used to measure the progress of protein folding and thus, a change from solubilized and intermediate states to the correctly folded form (Walther et al. 2014; Pathak et al. 2016). A full overview of available monitoring methods applied to refolding processes including advantages and limitations has been published elsewhere (Pauk et al. 2021).

SUMMARY OF THE INVENTION

The present invention is based on the development of a Digital Twin of the refolding process of proteins in a bioreactor, which reliably models the development over time of critical process parameters (CPPs), key performance indicators (KPIs) and critical quality attributes (CQAs) and of the refolding process. The interaction of an inclusion body refolding process with its digital counterpart allows (i) model-based experimental planning in process development for fed-batch refolding processes (ii) process monitoring, and (iii) optimal process control. This integrated approach does not only lead to an increase in productivity and recovery yield, but additionally can increase process robustness.
Thus, according to a first aspect the invention relates to a method for optimizing the production of a protein, comprising a refolding process, using a Digital Twin of the production process comprising the following steps:

- generating the Digital Twin of the production process by reproducing the refolding process and optionally further unit operations by means of mathematical models and known values of critical process parameters (CPPs), and process determinants selected from critical quality attributes (CQAs) and key performance indicators (KPIs) of the unit operations,
- varying the input of at least one CPP within a predetermined range in the Digital Twin and calculating predicted values or the development over time of at least one process determinant in the Digital Twin;
- determining the input of the CPP for which an optimal value or development over time of the process determinant is predicted in the Digital Twin as the optimally predicted CPP input; and
- adjusting the CPP input to the optimal predicted CPP input when performing the unit operation of the production process.

This method is preferably used iteratively in order to optimize a KPI of the process such as recovery yield or -space-time-yield or a CQA such as the concentration of the correctly folded native protein or other protein states. As shown in the examples, using the Digital Twin of the refolding process of LDH, it was possible to increase the recovery yield of native protein, i.e. from about 28 % to about 55 %.
Moreover, the predictive power of the Digital Twin optimized for the refolding process of an individual protein is used to reduce and counteract against process disturbances resulting from technical failure or feed stock variability. The proposed platform technology is used as an essential tool for quality assurance and validation requirements.
Thus, according to a second aspect, the invention relates to a method for producing a protein comprising a refolding process, wherein the refolding process is carried out in a bioreactor and is monitored and/or controlled by means of a Digital Twin of the refolding process, wherein the Digital Twin comprises a state-observer algorithm in combination with a model of the refolding process based mathematical models of the reaction kinetics and known values of CPPs and process determinants selected from CQAs and KPIs of the refolding process.
Monitoring of the process is conducted in real-time, combining available process analytical technology tools and state-estimation, in particular using a particle filter, inside the Digital Twin. With the Digital Twin it is possible to obtain information on the development over time of a single CQA or KPI over the whole process with only a few actual measurements of the CQAs or KPIs. Moreover, each CQA included in the model of the Digital Twin may be estimated without the necessity to measure each of the CQAs. The real-time state information in the Digital Twin may be utilized to drive the refolding process into an optimized direction via model-based process control based on the user's preferences.
The inventors have further developed a specific bioreactor, a so-called "refolding unit", with increased options for influencing the CPPs of the refolding process, thereby reducing the process development times. The bioreactor further allows an improved process monitoring as it contains an aseptic sampling port connected to an on-line or at-line measurement unit. Thus, according to a third aspect, the invention provides a bioreactor for the refolding process of proteins, comprising a process vessel, a temperature control, a gassing unit for the addition of one or more of $N_2$, $O_2$ and process air, at least five addition groups, one or more sensors and an aseptic sampling port connected to an on-line or at-line measurement unit for one or more process determinants.

According to a fourth aspect, the invention provides a method of refolding a protein using a bioreactor according to third aspect, comprising the monitoring of one or more process CQAs using an on-line or at-line measurement unit for the one or more process determinants, wherein the CQAs are selected from the concentration of solubilized protein, the concentration of intermediate state, the concentration of aggregated protein and the concentration of native protein, the concentration of misfolded protein, the concentration of the cofactor protein complex and the concentration of unbound cofactor.

Together with the Digital Twin, the bioreactor forms an improved bioreactor system for optimizing, monitoring and controlling the refolding processes of proteins from inclusion bodies. Thus, in a fifth aspect the invention provides a bioreactor system comprising a bioreactor according to the fourth aspect, a control system with a process control software, wherein the control system is connected to and control the temperature control, a gassing unit for the addition of one or more of $N_2$, $O_2$ and process air, at least five addition groups, a signal converter connected to the sensors of the bioreactor; and a Digital Twin of the refolding process, wherein the Digital Twin comprises a state-observer algorithm in combination with a model of the refolding process based mathematical models of the reaction kinetics and known values of CPPs and process determinants selected from CQAs and KPIs of the refolding process, implemented on a computer, wherein the Digital Twin is connected to the control system and the signal converter.

## FIGURES

Fig. 1       shows an overview of the Inclusion body refolding dynamics.

Fig. 2       shows a schematic depiction of an inclusion body downstream processing chain. Highlighted sections include critical unit operations.

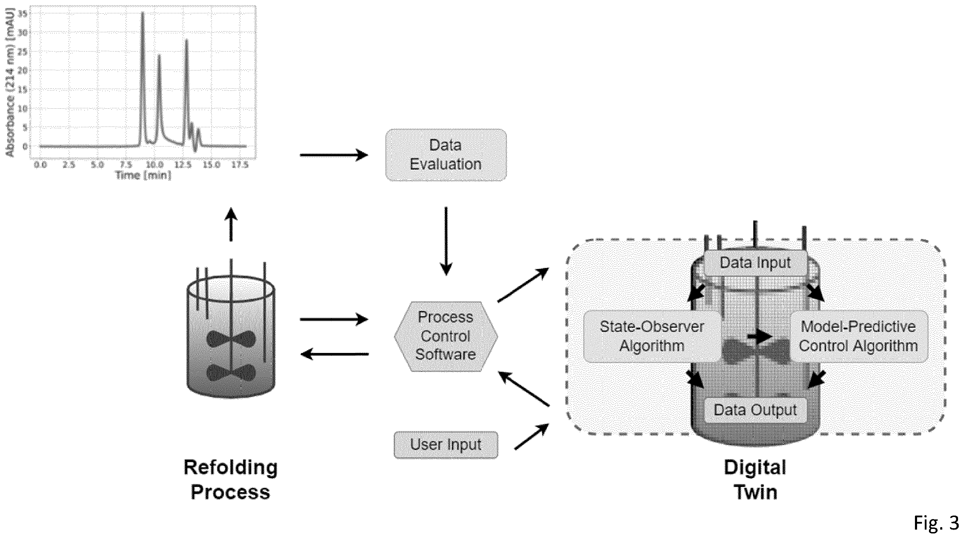

Fig. 3       Schematic representation of the interaction between the Digital Twin and a fed-batch refolding process via a process control software.

Fig. 4       shows real-time process monitoring of a fed-batch refolding process. State-estimation via particle filter of the total protein concentration (_), concentrations of the aggregates ( ....... ), native protein (_), and intermediate states ( ▬▬ ) and measurements of the native (□) and intermediate (◇) state used during the update step of the particle filter.

Fig. 5       shows a schematic overview of the model-based experimental design cycle.

Fig. 6       shows the results of a fed-batch refolding process with constant feeding. Measured concentrations of total protein (○), native state (□), aggregates (×), and intermediate states (◇). In Figure A simulations are based on the initial parametrized INA model and actual process parameters. Figure B shows simulations based on the parametrized adapted INA model with $c_{l,min}$ extension and actual process parameters. Vertical dotted lines indicate the start of accumulation of folding intermediates.

Fig. 7       shows the process design for a two-feed fed-batch system and a feed dilution process for analysis of the dependency of the refolding performance on the total protein concentration (......) and the GuHCl concentration (...) under consideration of the reactor volume (_).

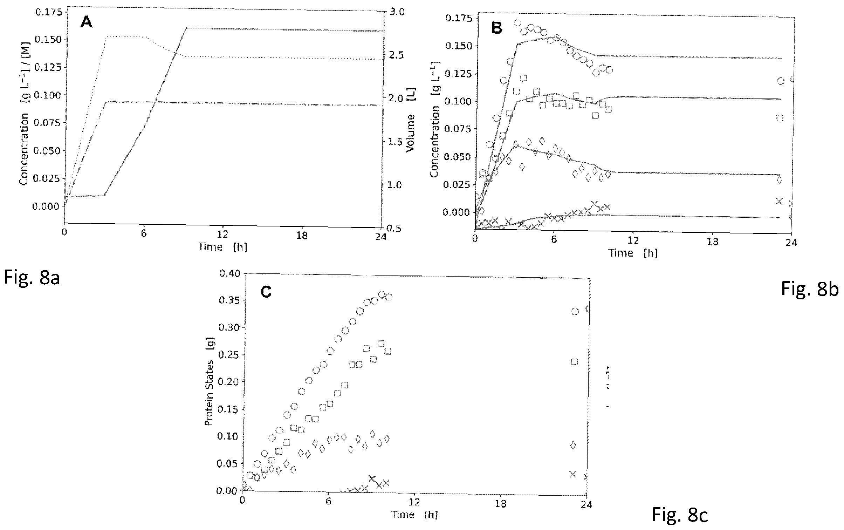

Fig. 8       shows the results obtained using the three-feed-system for identification of refolding and aggregation rates in a LDH fed-batch refolding process (see Example 6). Figure A shows the prediction of total protein concentration (......), GuHCl concentration (...), and changes in reactor volume (_) in a three-feed fed-batch process calculated based on the parametrized INA model. Figure B shows the measured concentrations of total protein (○), native state (□), aggregates (×), and intermediate states (◇) and simulations based on the parametrized model and actual process parameters. Figure C shows the total protein (○), native state (□), aggregates (×), and intermediate states (◇).

Fig. 9    shows the refolding rates over time according to state of the art (see Dong et al. 2004) and the adapted rate incorporating concentration of denaturing agent and total protein according to the invention.

Fig. 10    shows a flowchart of the particle filter of the digital twin used for the monitoring the refolding process in a bioreactor.

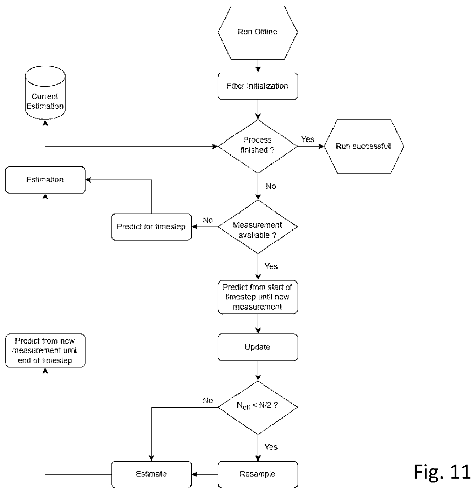

Fig. 11    shows a flowchart of the particle filter and the model predictive control and of the digital twin used for the controlling the refolding process in a bioreactor.

DETAILED DESCRIPTION OF THE INVENTION

A Digital Twin as used herein is a virtual representation that serves as the real-time digital counterpart of a physical object or process. In addition to generating a simulated representation, the Digital Twin can also be configured to communicate to a user or another machine via different communication channels. Furthermore, the Digital Twin may be configured to communicate with other Digital Twins in order to form an integrated network of twins, which simulate operations of different assets of at least one process or at least one product.

Definitions

According to the invention, a "critical quality attribute" (CQA) is a physical, chemical, biological, or microbiological property or characteristic that should be within an appropriate limit, range, or distribution to ensure the desired product quality.
As used herein, a "key performance indicator" (KPI) is a metric for the status of each production step.
As used herein, "a critical process parameter" (CPP) is a process parameter whose variability has an impact on a critical quality attribute and, therefore, should be monitored or controlled to ensure the process obtains the desired quality.
"Measuring a parameter", such as a CPP, a CQA or a KPI, as used herein may refer to the a direct measurement using a suitable sensor, e.g. the temperature via a temperature sensor or an indirect measurement where the parameter, in particular the CQA or KPI values are calculated from other physical measurements, such as protein concentrations from HPLC measurements or enzymatic assays. The indirect measurement is also referred to as measurement by soft sensors.
As used herein, a "process determinant" refers either to a KPI or to a CQA.
As used herein "process analytical technology" (PAT) includes the tasks of designing, analyzing and controlling production processes based on real-time monitoring of critical parameters including the CPPs and CQAs.
"Process monitoring", as used herein, is the description of the actual state of the process system, resulting from a combination of measurements, either direct or from soft sensors, and predictions from a state-observer, in order to follow the process in real-time, and to detect deflections of CPPs or process determinants in time.
"Unit operation" as used herein is a basic step in the production process, such as cell cultivation, solubilization of the inclusion body or the refolding process.
"State observer" or "state-observer algorithm" as used herein is a computer implemented mathematical model that provides the estimation of the internal states of a system.
A "cost function" as used herein has the general meaning as in mathematical optimization and decision theory. It is a function that maps an event or values of one or more variables onto a real number intuitively representing some "cost" associated with the event. An optimization problem seeks to minimize a cost function.

**Optimization of the production process**

According to a first aspect, the invention relates to a method for optimizing the production of a protein, comprising a refolding process, using a Digital Twin of the production process, comprising the following steps:

- generating the Digital Twin of the production process by reproducing the refolding process and optionally further unit operations by means of mathematical models and known values of critical process parameters (CPPs), and process determinants selected from critical quality attributes (CQAs) and key performance indicators (KPIs) of the unit operation;
- varying the input of at least one CPP within a predetermined range in the Digital Twin and calculating predicted values or the development over time of at least one process determinant in the Digital Twin;
- determining the input of the CPP for which an optimal value or development over time of the process determinant is predicted in the Digital Twin as the optimally predicted CPP input; and

- adjusting the CPP input to the optimal predicted CPP input when performing the production process in the bioreactor.

According to one embodiment, the predicted process determinant is a KPI. The KPI may be selected from recovery yield of native protein and space-time yield. According to one embodiment predicted process determinant is a CQA selected from the solubilized protein concentration, intermediate state concentration, aggregated protein concentration, native protein concentration, misfolded protein concentration, cofactor protein complex concentration and unbound cofactor concentration. These concentrations can be measured separately with a variety of methods.

The methods include circular dichroism (CD) spectroscopy, fluorescence spectroscopy, infrared (IR) spectroscopy, Raman spectroscopy, nuclear magnetic resonance (NMR) spectroscopy, reversed-phase high-performance liquid chromatography (HPLC) size exclusion HPLC. Further methods are the bicinchonomic assay (BCA) assay, in particular for the determination of the total protein concentration, and turbidity measurements for the content of aggregates. In case the refolded protein is an enzyme, enzymatic assay specific for the activity of the enzyme can be used as a measure for the concentration of native protein. In case of the Lactate dehydrogenase (LDH) the LDH Activity Assay kit can be used. In this kit, LDH reduces NAD to NADH, which is specifically detected by colorimetric (450 nm) assay. Other test methods use the oxidation from NADH to NAD+ measured at a wavelength of 340 nm.

The yield is the quotient of the quantity (or concentration) of the native protein at a given time point (t) and the quantity (or concentration) of the total protein at the start of the refolding process (t = 0). The space-time yield (STY) is calculated as the quotient of the quantity of the native protein at a given time point (t) and the product of the volume of the refolding solution and the elapsed time or the quotient of the concentration of the native protein and the time. The unit of the STY is mol $l^{-1}$ $h^{-1}$ or g $L^{-1}$ $h^{-1}$.

Depending on the KPI or CQA, the optimal value may be a maximum or minimum. Generally, the optimal value of the recovery yield, the space-time yield is a maximum and the native protein is a maximum. In contrast, the optimal value of the solubilized protein concentration, intermediate state concentration, aggregated protein concentration, cofactor protein complex concentration, and misfolded protein should be a minimum.

There is a variety of parameters influencing the refolding process. The CPP may be a quality parameter like the type of the denaturing agent or the type of the redox partner. Alternatively, the CPP may be a quantity parameter like the pH, temperature or protein feed rate. For the case that the CPP is a quality parameter, the input is the quality such as the specific denaturing agent. In case the CPP is a quantity parameter such as the protein feed, the input may be a constant value or may be defined by a function over time.

According to one embodiment, the CPPs for the refolding process are selected from: type of buffer, buffer concentration, ionic strength, pH, temperature, pressure, mechanical energy input, starting concentration of protein, mode of addition of the protein, protein feed rate, mode of addition of the refolding buffer, buffer feed rate, protein addition time, redox potential and redox partners, type of co-factors, concentration of co-factors, type of denaturing agent, feed of denaturing agent, concentration of denaturing agent, type of refolding technique, duration of the refolding process. As shown in the examples, it was possible by adjusting the protein feed rate and the feed of the denaturing agent to significantly increase the recovery yield of the native protein.

The refolding buffer used for a given protein of interest is customized to the refolding requirements/kinetics of that protein. Refolding buffers are known in the art and commercially available. Typical buffer components are denaturing agents, EDTA, salts, and refolding additives like L-arginine or sugars. Denaturing agents are for example guanidine hydrochloride, or urea. Redox partners are for example reduced and oxidized glutathione (GSH/GSSG), cysteine/cystine, cysteamine/cystamine and may be included in the refolding buffer and/or added separately. The refolding buffer may further comprise detergents, such as N-lauroylsarcosine, alcohols, such as e.g. N-propanol), low molecular weight thiols, such as dithiothreitol (DTT) or β-mercaptoethanol.

The bioreactor used in the process may be a batch reactor, a fed-batch reactor a continuous reactor. The batch reactor is the generic term for a type of vessel widely used in the process industries. A typical batch reactor consists of a storage tank with an agitator and integral heating/cooling system. A batch reactor is filled with reactants at the start (and the reaction proceeds. Fed-batch reactors operate like batch reactors but are modified to allow reactant addition in time. Continuous reactors carry material as a flowing stream, i.e. reactant addition and product removal in time. Reactants, in case of the refolding process the solubilized protein and supplements, such as denaturing agents, redox agents or the refolding buffer, are continuously fed into the reactor and the product. Continuous reactors are used for a wide variety of chemical and biological processes within the chemical and pharmaceutical industries and are available variety of shapes and types of machine. Fed-batch and continuous reactors are preferred because these reactor types allow more process parameters, in particular CPPs to be influenced. Preferably, the bioreactor is a fed-batch reactor.

The predetermined ranges of each of the CPPs are defined by physical, economic or legal constraints. Examples of physical constraints are the maximal pump rates of the specific pump heads used for feeding the solubilized protein or other components, the maximal solubility of a specific reagent, freezing temperatures and temperatures that start to denaturize the protein, the maximal filling volume of the reactor, the maximal solubility of $O_2$ in the buffer. Examples of economic constraints are the energy consumption of the process, the reactor size or the cost of the used additives. A high feed rate may consume too much energy for an economically reasonable process. Similarly, the dilution of the protein is limited by the size of the reactor that is economically reasonable. Examples of legal constraints are the maximal amount of host cell proteins, or the maximal amount of endotoxins from the host organism, in particular *E. coli*.

In order to further optimize the process, after the adjustment of the CPP, the actual value of the CQA or KPI may be measured. According to one embodiment, the method comprises the further steps of measuring the value or development over time of the process determinant and, determining the deviation of the measured value from the predicted value of the process determinant or the deviation of the measured development over time from the predicted development over time of the process determinant.

The deviation is a measure how well the model fits the actual process. There are different method available for quantifying the deviation. The weighted residual sum of squares (WRSS) indicates the total error of the fit. The normalized root mean squared error (NRMSE) indicates the error of a certain process parameter, such as a CPP or CQA. Various other criteria which, in addition to the goodness of fit, also include the complexity of the model and can therefore reduce a possible overfitting (AIC, BIC, etc.). A visual control and extrapolation of the simulation data is also useful, because different models can show the same performance criteria although different curves can occur. According to one embodiment, the deviation is the NRMSE.

The deviation is useful for deciding how to continue with the process. If the deviation is below a threshold it shows that the model is sufficiently accurate. The deviation threshold depends on the type of CQA or KPI. Generally, the deviation threshold of the NRMSE should at most 15 %. Preferably, the NRMSE threshold is at most 10 %. More preferably, the NRMSE threshold is at most 5 %. Accordingly, the model may then be used to optimize a second CPP of the process. Thus, the folding process can be iteratively optimized. According to one embodiment, the method comprises the further steps of varying the input of at least one second CPP within a predetermined range in the Digital Twin, and calculating the predicted value or predicted development over time of the process determinant in the Digital Twin; determining the input of the second CPP for which an optimal predicted value of the process determinant is calculated in the Digital Twin as the optimally predicted second CPP input; and setting the second CPP to the optimally predicted second CPP input when performing the refolding process.

On the other hand, if the deviation is greater than the threshold, it is preferred to adjust the model to better reflect the real refolding process. Thus, according to one embodiment, if the deviation is greater than a threshold value, the method contains the further step of adjusting the mathematical models in the Digital Twin to match the pair of values of the CPP and the measured process determinant.

As shown in Figure 2, the typical process of protein production including a refolding process starts with the cultivation of the bacterial cells, followed by disruption of the bacterial cells, isolation of the including bodies, washing of the inclusion bodies and solubilizing the protein. The solubilized protein is then added into the refolding reactor. Each of these unit operations of the production process can also be modeled in the Digital Twin. By including further unit operations into the Digital Twin, it is possible to directly predict the influence of CPPs of these unit operations on the outcome of the refolding process.

According to one embodiment, the Digital Twin includes a model of the solubilization process. The CPPs for the solubilization process are for example type of buffer, buffer concentration, ionic strength, pH, temperature, pressure, mechanical energy input, starting concentration of protein, type of denaturant, concentration of denaturant, type of co-factors (additives), concentration of co-factors, oxidant concentration before solubilization, protein concentration, denaturant concentration. According to one embodiment, the Digital Twin consists of a model of refolding process and a model of the solubilization process.

According to one embodiment, the Digital Twin includes a model of the cell cultivation. Relevant CPPs for cell cultivation are, for example, the expression organism, in particular the E. coli strain, the type of culture medium, the substrate concentration, pH, temperature, dissolved oxygen, dissolved $CO_2$, the concentration of expression organism, the mechanical energy input, the viscosity, the volume of culture medium and total volume of bioreactor, the induction time, the concentration of induction agent, the total cultivation time, the total time of induced production, the method of cell harvest/separation of cells from the culture broth. According to one embodiment, the Digital Twin consists of a model of refolding process and a model of the cell cultivation.

According to one embodiment, the Digital Twin includes a model of the inclusion body isolation. Relevant CPPs for inclusion body isolation are, for example, the type of cell disruption process, the components of the lysis buffer and their concentrations, the number of disruption cycles, the duration of a disruption cycle, the disruption pressure in case disruption by pressure, amplitude, cycle and total sonication time if disruption by sonication. According to one embodiment, the Digital Twin consists of a model of refolding process and a model of the inclusion body isolation.

According to one embodiment, the Digital Twin includes a model of the washing the inclusion bodies. Relevant CPPs for the washing the inclusion bodies for example the components of the wash buffer and their concentrations, the pH, the number of wash cycles, the duration of a wash cycle, the temperature, the method of resuspension, the speed and total time of centrifugation between wash cycles. According to one embodiment, the Digital Twin consists of a model of refolding process and a model of the washing the inclusion bodies. According to one embodiment, the Digital Twin consists of a model of refolding process, the solubilization process and a model of the washing the inclusion bodies. According to one embodiment, the Digital Twin consists of a model of refolding process, the solubilization process, a model of the washing the inclusion bodies and inclusion body isolation.

According to one embodiment, the predicted process determinant is a KPI. The KPI may be selected from recovery yield of native protein and space-time yield. According to one embodiment, the predicted process determinant is a CQA selected from solubilized protein concentration, intermediate state concentration, aggregated protein concentration, native protein concentration, misfolded protein concentration cofactor protein complex concentration, and unbound cofactor concentration.

Theoretically, the refolding process of any protein can be modelled. Therefore, the protein of which the production process is optimized can be any protein. According to one embodiment, the protein forms inclusion bodies in recombinant expression.

The size of the protein is in principle not limited. However, the complexity of the refolding process increases with the size. According to one embodiment, the protein has an amino acid length of at most 1000 amino acids. According to a preferred embodiment, the protein has at most 700 amino acids. According to a more preferred embodiment, the protein has at most 500 amino acids. In particular, the protein has at most 350 amino acids.

Moreover, the mechanistic model becomes more complex with an increasing number of disulfide bridges in the protein. According to one embodiment, the protein has at most 10 disulfide bridges. The protein may have for example 0, 1, 2, 3, 4, 5, 6, 7, 8, 9, or 10 disulfide bridges. According to a one embodiment, the protein has at most 4 disulfide bridges. According to one embodiment the protein has 2 disulfide bridge. According to one embodiment the protein has no disulfide bridges.

Furthermore, the mechanistic model of the refolding and assembly to the active protein is dependent on the quaternary structure. According to one embodiment, the proteins is a single amino acid chain. However, the protein may be a heteromer of different or a homomer of identical subunits. The protein may have, for example, 2, 3, 4, 5, 6, 7, 8, 9, or 10 subunits. According to a one embodiment, the protein has at most 6 subunits. According to one embodiment the protein has at most 4 subunits. According to one embodiment the protein has at most 2 subunits.

The protein may have one or more cofactors. Cofactors are essential components of many proteins to attain biological activity. However, some cofactors also play a role in protein folding, in particular stabilizing the native protein or interacting specifically with the unfolded polypeptide. The cofactor may be an inorganic ion, such as an iron, a copper, a magnesium or a manganese ion, or a complex organic molecules selected from a vitamin or a non-vitamin. Examples are $NAD^+$, thiamine pyrophosphate (TPP), flavin adenine dinucleotide (FAD), biotin, and lipoamide. The protein may have one co-factor per subunit.

The protein may be for example selected from lactate dehydrogenase (LDH), galactose oxidase (GalOx), horse radish peroxidase (HRP), human insulin, interferon α2a, filgrastim (G-CSF analog), interferon α2a, glucagon, long-acting insulin glargine, human parathyroid hormone and human granulocyte stimulating factor (GCSF).

The LDHs catalyze the conversion of lactate to pyruvate and back, as it converts $NAD^+$ to NADH and back. LDHs do not involve a cofactor. There are different LDH proteins differentiated by the number of copies of the two subunits LDHA and LDHB. LDH1 is composed from four LDHB subunits; LDH2 contains three LDHB subunits and one LDHA; LDH3 has two LDHB/LDHA subunits; LDH4 possesses one LDHB subunit and three LDHA subunits; while LDH5 is composed from four LDHA subunits. LDH1 is a protein of 320 amino acids and does not contain disulfide bridges. According to one embodiment, the LDH is LDH1. According to one embodiment, the LDH is from *Lactobacillus plantarum* (Uniprot: P56512 (LDH1_LACPL)).

GalOx is an enzyme that catalyzes the oxidation of D-galactose in some species of fungi using copper ions as cofactor. GalOX comprises 680 amino acids, two disulfide bridges and one thioether crosslink.

According to one embodiment, the protein is a recombinant protein produced in bacteria as host cells, in particular in *E.coli*

According to one embodiment of the method of the first aspect the protein is LDH, the KPI is the recovery yield of native protein, and the CPP is the protein feed rate and the denaturant feed rate.

The mathematical models used to reproduce the refolding process are in particular mechanistic models of the reaction kinetics of the refolding of the protein. The mechanistic models may be deterministic and/or stochastic models. The models may additionally include information on the protein structure.

The mechanistic model used to describe the refolding process preferably consists of a system of differential equations based on the fed-batch adaptations of original protein refolding models with an assumed aggregation reaction of second order or higher. It consists of four protein states S, I, N and A.

According to one embodiment, the underlying mathematical model of the reaction kinetics is a four-state model with the states solubilized protein (S), intermediate state (I), aggregated protein (A) and native protein (N). According to one embodiment the reaction from S to I is a rapid reaction determined by the reaction rate $k_i$. The reaction from I to N may be a first order reaction and determined by the reaction rate $k_n$. The reaction from I to A may be a higher order reaction determined by the reaction rate $k_a$.

Folding intermediates are formed in the instant solubilized protein is diluted into the refolding buffer (Figure 2). Thus, the model is reduced to three protein states (Equations 1-3),

$$\frac{dc_I}{dt} = -(k_n \cdot c_I + k_a \cdot c_I{}^2) + \frac{\sum_{k=1}^{r} F_{R,k} \cdot c_{SR,k}}{V} - \frac{\sum_{k=1}^{r} F_{R,k}}{V} \cdot c_I \qquad (1)$$

$$\frac{dc_N}{dt} = k_n \cdot c_I - \frac{\sum_{k=1}^{r} F_{R,k}}{V} \cdot c_N \qquad (2)$$

$$\frac{dc_A}{dt} = k_a \cdot c_I{}^2 - \frac{\sum_{k=1}^{r} F_{R,k}}{V} \cdot c_A \qquad (3)$$

as well as states for denaturant concentration and reaction volume (Equations 4 and 5).

$$\frac{dc_D}{dt} = \frac{\sum_{k=1}^{r} F_{R,k} \cdot c_{DR,k}}{V} - \frac{\sum_{k=1}^{r} F_{R,k}}{V} \cdot c_D \qquad (4)$$

$$\frac{dV}{dt} = \sum_{k=1}^{r} F_{R,k} \qquad (5)$$

Here, $r$ denotes the number of feeds, $k$ the reservoir counter, $F_{R,k}$ the feed rate of feed $k$, $c_{SR,k}$ the concentration of solubilized protein in reservoir $k$ and $c_{DR,k}$ the concentration of the denaturant in reservoir $k$. The model is based on the assumption, that all feeds solely consist of solubilized protein and the refolding vessel is protein free prior to process start.

Of note, in case of a continuous reactor, equation (5) is modified by the flowrate of the mass stream leaving the reactor ($F_{out}$)

$$\frac{dV}{dt} = \left(\sum_{k=1}^{r} F_{R,k}\right) - F_{out} \qquad (5a)$$

The reaction rates of the refolding and aggregation reaction are both dependent on the denaturant concentration,

$$k_m = a_m \cdot (1 + c_D)^{b_m} \qquad (6)$$

with m = $n$, $a$, the proportional model parameter $a_m$, and the model parameter $b_m$ to penalize the reaction rate with increasing denaturant concentration.

The setup of the process model generally followed the scheme described in Daume et al. (2020). The parametrization was achieved by global optimization using a genetic algorithm and further refined with the Nelder-Mead Simplex algorithm deployed in MATLAB (The MathWorks Inc., Natick, USA). Objective function for both algorithms was the weighted residual sum of squares (WRSS), where the error between measurement ($c_{i,j}$) and estimation ($\hat{c}_{i,j}$), weighted by variances of measurement errors ($\sigma_{i,j}$), was squared and summed for every measurement point $j$ and state $i$

$$min(WRSS(\theta)) = \sum_{i=1}^{n} \sum_{j=1}^{d} \left(\frac{c_{i,j} - \hat{c}_{i,j}}{\sigma_{i,j}}\right)^2 \qquad (7)$$

The optimized parameter set $\theta$ was gained by minimizing the WRSS. Validation of the fit of each model state was performed by calculating the state error with the normalized root mean squared error (NRMSE).

$$NRMSE(c_i) = \frac{\sqrt{\frac{1}{d} \cdot \sum_{j=1}^{d} \left(c_{i,j} - \hat{c}_{i,j}\right)}}{\max(c_i) - \min(c_i)} \tag{8}$$

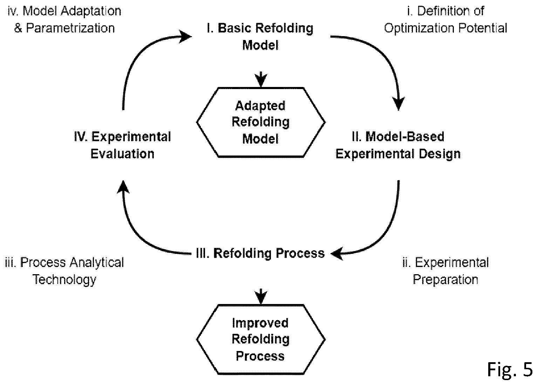

As shown in the examples, available mechanistic process models were further optimized following the proposed model-based experimental design cycle (Figure 5). This cycle includes scanning the design space for possible optimization potential, preparing, and conducting the actual experiment, PAT, and data evaluation after the experiment and finally parametrization and adaptation of the model.

Based on the experimental results of Example 5, the model was further adapted. A model parameter $c_{I,min}$ is scaled by the current total and expected maximal total protein concentration ($c_P$, $c_{P,max}$) and is incorporated into the model only in the reaction part of the differential equations (Equation 1-3) but not into the dilution term, yielding the adapted model (Equations 9-12).

$$c_{I,react} = c_I - c_{Imin} \cdot \frac{c_P}{c_{P,max}} \tag{9}$$

$$\frac{dc_I}{dt} = -\left(k_n \cdot c_{I,react} + k_a \cdot c_{I,react}^2\right) + \frac{\sum_{k=1}^{r} F_{R,k} \cdot c_{SR,k}}{V} - \frac{\sum_{k=1}^{r} F_{R,k}}{V} \cdot c_I \tag{10}$$

$$\frac{dc_N}{dt} = k_n \cdot c_{I,react} - \frac{\sum_{k=1}^{r} F_{R,k}}{V} \cdot c_N \tag{11}$$

$$\frac{dc_A}{dt} = k_a \cdot c_{I,react}^2 - \frac{\sum_{k=1}^{r} F_{R,k}}{V} \cdot c_A \tag{12}$$

These optimized models describe the process sufficiently well to use the Digital Twin in process monitoring in particular for state estimation. Suitable software to calculate model observability is available for non-linear process models, such as the STRIKE-GOLDD toolbox for MATLAB developed by Villaverde et al. (2019).

According to one embodiment, the mathematical model in the Digital Twin is based on one or more of equation (1), equation (2), equation (3), equation (4), equations (5), equation (5a), equation (6), equation (7), equation (8), equation (9), equation (10), equation (11) and equation (12). According to one of the embodiments, the mathematical model in the Digital Twin is based on equations (1) to (5). According to one of the embodiments, the mathematical model in the Digital Twin is based on equations (4) and (5) and equations (9) to (12).

Monitoring of the refolding process

According to a second aspect, the invention provides a method for producing a protein comprising a refolding process, wherein the refolding process is carried out in a bioreactor and is monitored by means of a Digital Twin of the refolding process, wherein the Digital Twin comprises a state-observer algorithm in combination with a model of the refolding process based on mathematical models of the reaction kinetics and known values of the CPPs and process determinants selected from CQAs and KPIs of the refolding process.

The method comprises the steps of:

- feeding the input of CPPs and process determinants selected for the refolding process into the Digital Twin and initializing the state-observer algorithm, wherein the initializing comprises defining a set of process determinants and optionally CPPs as the initial condition of the process; and
- starting the refolding process in the bioreactor with the selected input of the CPPs and process determinants.

With these steps, the model of the process is aligned with the actual settings in the bioreactor at the start of the process.

The process determinants and optionally CPPs in the set defining the condition of the process are also referred to as model states. According to one embodiment, the set of process determinants and optionally CPPs defined as the initial condition include at least three parameters. This set of defines the condition of the process during the course of the process. The set may include 3, 4, 5, 6, 7, 8, 9, 10, 11, 12, 13, 14, 15, 20, or 25 parameters. According to one embodiment, the refolding process is run as a batch process and the set of process determinants includes four process determinants. According to one embodiment, the set includes at least five parameters. According to one embodiment, the refolding process is run as a fed-batch process and the set includes six parameters. According to one embodiment, the set includes at least seven parameters.

State-observer algorithms according to the invention may be Kalman filters and particle filters. Contrary to Kalman filters the particle filter is able handle even strong non-linearities in the process model and measurement functions. It is Monte Carlo based and, therefore, relies on many simulations and consequently increased computational power.

According to one embodiment, the state observer algorithm is a particle filter algorithm. The particle filter is initialized by distributing a number of sampling points (particles) either uniformly in the complete design space of the process or as Gaussian distribution around a known initial process condition, i.e. a defined set of model states, which are process determinants and optionally CPPs of the system. According to one embodiment, the number of particles at 100. The number of particles may for example 100, 200, 300, 400, 500, 600, 700, 800, 900, 1,000, 2,000, 3,000, 4,000, 5,000, 6,000, 7,000, 8000, 9000, or 10,000. According to one embodiment, the number of particles is in the range at least 200. According to one embodiment, the number of particles is in the range at least 500.

Thus, each particle contains the values of the set of process determinants and optionally CPPs defining the condition of the process. Each particle has an associated weight, which is equally distributed during the initialization stage.

The one or more process determinants may be CQAs or KPIs. The CQA may be selected from the concentration of solubilized protein, the concentration of intermediate state, the concentration of aggregated protein, the concentration of native protein, concentration of the misfolded protein, the concentration of the protein-cofactor complex and the concentration of the unbound cofactor. The KPI may be selected from the yield and the space-time-yield.

The CPPs may be, for example, selected from pH, temperature, pressure, mechanical energy input, volume of the refolding solution, protein feed rate, protein addition time, concentration of redox buffers and feed rate of the denaturing agent, denaturing agent addition time and concentration of the denaturing agent. Measurements of the CPPs may be performed using corresponding sensors in the bioreactor. According to one embodiment, the CPPs are constantly measured during the process.

According to one embodiment, the set of parameters defining the condition includes one or more of the concentration of solubilized protein, the concentration of intermediate state, the concentration of aggregated protein, the concentration of native protein, the feed of the denaturing agent and the volume of the refolding solution.

According to one embodiment, the set of parameters defining the condition includes, in particular consists of, the concentration of solubilized protein, the concentration of intermediate state, the concentration of aggregated protein, the concentration of native protein, the feed of the denaturing agent and the volume of the refolding solution.

According to one embodiment, the method comprises measuring one or more of the process determinants of the set defining the condition of the process in the bioreactor. CQAs may be measured using the methods described above.

According to one embodiment, after the lapse of a monitoring time step, the Digital Twin performs the step of predicting the temporal evolution of the set of process determinants and optionally CPPs defining the process condition during the monitoring time step based on the model of the refolding process. In case, the result of a measurement of a process determinant is available in the monitoring time step, the measured value is fed into the Digital Twin.

According to one embodiment, the state observer is a particle filter and the step of predicting includes:

a) predicting the temporal evolution of each particle based on the model of the refolding process;
b) updating the weight of each particle based on the error between the predicted position and the measured values;
c) resampling of the particles; and
d) estimating the process condition based on the calculation of the weighted mean and covariance of the particles distribution.

The steps b) and c) are only carried out if measured values of one or more process determinants or CPPs of the set defining the process condition are fed into the Digital Twin within the monitoring time step.

In the prediction step a), the trajectory of each particle is simulated via the model with the current estimation as initial condition for a given time step. Random white noise may be added to every model parameter prior to the simulation of each particle to model the probability distribution of the model parameters.

For the case that measured values of one or more process determinants or CPPs of the set defining the process condition are fed into the Digital Twin within the monitoring time step, the steps b) updating and c) resampling are carried out. In particular, the Digital Twin may predict the temporal evolution of each particle from the start of the monitoring step to the time point of the measurement, update the weight of each particle, resample the particles, and estimate the process condition at the time of the measurement, and then perform a second round of prediction and estimation from the time point of measurement to the end of the monitoring time step.

In the updating step b), the particle weights are scaled based on the error between the particle and measurement. The error of each particle between the current prediction and the measured value is calculated for all states with available new measurements. This summed error is used to scale the weights associated to the particles and normalized afterwards. Consequently, particles close to the measurements have their weights increased and particles far from the measurements have their weights decreased, hence updating the believe that these particles describe to the true state of the process.

In the resampling step c), the particles are resampled when the number of effective particles (see Equation 13) is too low (generally below half the number of particles $N$),. Resampling methods include multinomial resampling, residual resampling, stratified resampling and systematic resampling. The preferred resampling method is systematic resampling. Aim of this process is to replace particles with low weights by duplications of particles with higher weights and thus increasing the accuracy of the filter. Particles are drawn from the distribution of the weights, thus resulting in high probabilities to replace particles with low weights by duplications of particles with high weights.

$$N_{eff} = \frac{1}{\sum_{p=1}^{N} w_p{}^2} \qquad (13)$$

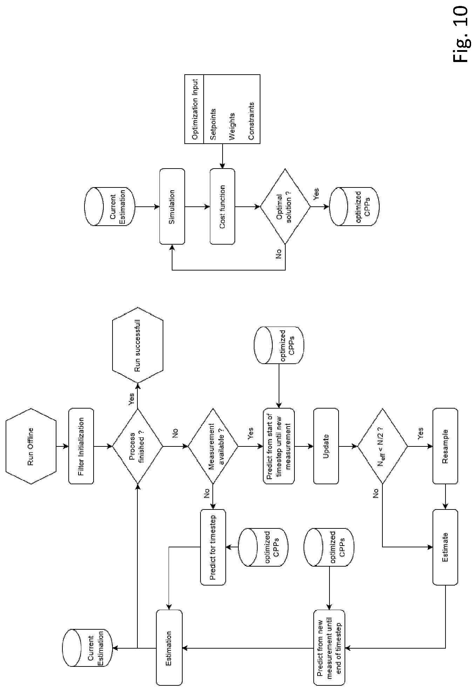

Aim of this process is to replace particles with low weights by duplications of particles with higher weights and, thus, increasing the accuracy of the filter. In particular, as shown in Figure 10, the particles are resampled if $N_{eff} < \frac{N}{2}$.

The predefined monitoring time step may be in the range of 30 sec to 60 min. The monitoring time step may be for example 30 sec, 1 min, 2 min, 4 min, 6 min, 8, min, 10 min, 15 min, 20 min, 25 min, 30 min, 35 min, 40 min, 45 min, 50 min, 60 min. Preferably, the monitoring time step is in the range of 1 min to 20 min. More preferably, the monitoring time step is in the range of 1 min to 10 min. Most preferably, the monitoring time step is in the range of 2 min to 5 min. According to one embodiment, the step of predicting is iteratively repeated after the lapse of each monitoring time step until the end of the process.

According to one embodiment of the method of optimizing the refolding process, the Digital Twin comprises a state-observer algorithm in combination with a model of the refolding process as defined above for the method of monitoring the refolding process. In this regard, the method of optimizing the refolding process may comprise a the refolding process being carried out in a bioreactor and monitored by means of the Digital Twin including the state-observer algorithm, in particular particle filter algorithm.

Controlling of the refolding process

Full state monitoring enables the usage of model-based control techniques, such as model predictive control (MPC). Here, the model is used to calculate the optimal system input to follow a given trajectory in an online optimization process.

According to an alternative of the second aspect, the invention provides a method for producing a protein comprising a refolding process, wherein the refolding process is carried out in a bioreactor and is controlled by means of a Digital Twin, wherein the Digital Twin comprises a state-observer algorithm in combination with a model predictive controller and a model of the refolding process based on mathematical models of the reaction kinetics and known values of the CPPs and process determinants selected from CQAs and KPIs of the refolding process.

The method, in which the Digital Twin controls the refolding process comprises the same steps as defined above for the method in which the Digital Twin monitors the refolding process. According to one embodiment, the method further comprises the further step of defining a set point for at least one process determinant of the defined set.

According to one embodiment, at the end of each monitoring time step the following steps are carried out:

    d. calculating in the Digital Twin the predicted temporal evolutions of at least one process determinant for a current and alternative inputs of one or more CPPs, wherein the calculation is based on the process determinant value predicted at the start of the prediction horizon and optionally the measured value of the at least one process determinant;

    e. determining which of the temporal evolutions of the at least one process determinant minimizes the cost function based on the set point of the at least one process determinant, wherein this is defined as the optimal temporal evolution; and

    f. setting the one or more CPPs to the input associated with the optimal temporal evolution.

According to one embodiment, the process is a batch process, the process determinants of the set defining the process are the concentration of solubilized protein, the concentration of intermediate state, and the concentration of aggregated protein and the process determinant with a defined set point is the yield.

According to one embodiment, the process is a fed-batch process and the process determinants of the set defining the process are the concentration of solubilized protein, the concentration of intermediate state, the concentration of aggregated protein, the feed of the denaturing agent and the volume of the refolding solution.

According to one embodiment, the duration of the prediction horizon is at least twice the duration of the monitoring time step. The prediction horizon may be for example at least twice, at least three times, at least four times, at least five times, at least six times, at least seven times, at least eight times, at least nine times, at least ten times, at least 15 times, at least 20 fold the monitoring time step. According to one embodiment, the prediction horizon is in the range of two times to 50 times the monitoring time step. According to one embodiment, the prediction horizon is in the range of three times to 25 times the monitoring time step. According to one embodiment, the prediction horizon is in the range of 5 times to 15 times the monitoring time step.

According to one embodiment, the inputs of one or more CPPs are values or temporal evolutions. According to one embodiment, the temporal evolutions of the one or more CPPs may be limited in time to a specific control horizon. This limitation reduces the computation time. According to one embodiment, the control horizon of the one or more CPPs is 20 times the monitoring time step. Preferably, the control horizon is at most 10 times, at most four times, at most eight times, at most six times, at most four times, the monitoring time step.

The alternative inputs of each of the CPPs are restricted by physical, economic and/or legal constraints as defined above. Examples of physical constraints are the maximal pump rates of the specific pump heads used for feeding the solubilized protein or other components, the maximal solubility of a specific reagent, freezing temperatures and temperatures that start to denaturize the protein, the maximal filling volume of the reactor, the maximal solubility of $O_2$ in the buffer. Examples of economic constraints are the energy consumption of the process, the reactor size or the cost of the used additives. A high feed rate may consume to much energy for an economically reasonable process. Similarly, the dilution of the protein is limited by the size of the reactor that is economically reasonable. Examples of legal constraints are the maximal amount of host cell proteins, or the maximal amount of endotoxins from the host organism, in particular *E. coli.*

According to one embodiment, set points for more than one process determinant are defined. By way of example, set points for the yield and the space-time yield are defined. According to one embodiment, the set points of the process determinant are weighted differently. For example, the yield set point may have double the weight of the set point of the space-time yield.

The software of the Digital Twin may be in the form of a simulation program and is preferably implemented in a computer. This computer preferably contains a graphical user interface to visualize the monitoring for a user. For the control method, the computer is connected to at least one control unit of the bioreactor.

With the Digital Twin, the refolding process of any protein as defined above can be monitored or controlled.

Bioreactor

According to the third aspect of the invention provides a bioreactor for the refolding process of proteins. The bioreactor comprises a process vessel, a temperature control, a gassing unit, at least five addition groups, one or more sensors and an aseptic sampling port connected to an on-line or at-line measurement unit.

The process vessel may have a volume of at least 0.5 L. According to one embodiment, the volume of the process vessel is at least 1 L. For mixing, the process vessel may contain a magnetic driven impeller or impeller with a double mechanical seal.

The one or more sensors for monitoring CPPs inside the process vessel are selected from a temperature sensor, pressure sensor, pH sensor, dissolved oxygen (DO) sensor, and a gas flow sensor. Each sensor is intended to be in communication with a computer-implemented control system (e.g. a computer) for calculation and control of various parameters and for display and user interface.

The on-line or at-line measurement units allows measuring one or more CQAs selected from the concentration of solubilized protein, the concentration of intermediate state, the concentration of aggregated protein and the concentration of native protein. The on-line or at-line measurement units may be selected from CD spectrometer, fluorescence spectrometer, an IR spectrometer, a Raman spectrometer, an NMR spectrometer, a reversed-phase HPLC system or size exclusion HPLC system.

Temperature control units are known in the art. The temperature control units generally contain a heating and a cooling function. Gassing units for bioreactors are known in the art. The gassing unit is for the addition of one or more of $N_2$, $O_2$ and process air.

Addition groups according to the invention at least contain pumps, balances for accounting and bottles for solution storage. According to one embodiment of the bioreactor the addition groups are for the addition of the solubilized protein in solubilization buffer, an acid, a base, a redox buffer, and the refolding buffer and denaturing agent in refolding buffer. With an increasing number of addition groups, there are more options to control the refolding process. According to one embodiment, the bioreactor comprises at least six addition groups. Preferably, the bioreactor comprises at least seven addition groups.

**Method of refolding**

According to fourth aspect the invention provides a method of refolding a protein using a bioreactor according to the fourth aspect. The method comprises the monitoring of one or more process determinants using an on-line or at-line measurement unit for the one or more CQAs, wherein the CQAs are selected from the concentration of solubilized protein, the concentration of intermediate state, the concentration of aggregated protein the concentration of native protein, the concentration of misfolded protein and the concentration of cofactor. Preferably, the CQAs are selected from the concentration of solubilized protein, the concentration of intermediate state, the concentration of aggregated protein and the concentration of native protein.

**Bioreactor System**

According to a fifth aspect the invention provides a bioreactor system comprising

- a bioreactor according to the fourth aspect,
- a control system with a process control software, wherein the control system is connected to and controls the temperature control, a gassing unit for the addition of one or more of $N_2$, $O_2$ and process air, and the at least five addition groups of the bioreactor,
- a signal converter;
- connected to the sensors of the bioreactor; and
- a Digital Twin of the refolding process, wherein the Digital Twin was generated by mapping the refolding process by means of mathematical models of the reaction kinetics and known values of the CPPs and process determinants selected from CQAs and KPIs of the refolding process, implemented on a computer, wherein the Digital Twin is connected to the control system.

A signal converter according to the invention is a physical device to connect sensors. The signal converter enable the exchange of data between a sensor or any other equipment and the advanced process control software. It processes analog or digital input signals from a physical device and translates them into the desired output signal. The signal converter consists of several types of converters depending the type of the input signal. It may also include an amplifier for the amplification of the signals. The whole signal converter is built in a GMP compliant stainless steel box. The device provides several ports for connecting the physical devices to the signal converter.

A further possible component of the bioreactors system is a human machine interface (HMI). The bioreactor system may further contain an electrical cabinet in which the control system is integrated. The control system particularly the contains a process control software and/or a human machine interface (HMI).

According to one embodiment, the bioreactor system contains a process vessel, seven addition groups including pumps, balances for accounting and bottles for solution storage; a gassing unit with mass flow controllers; electrical cabinet with control system; a human machine interface (HMI), a process control software, an external temperature control unit, a steam generator; an external pump for cleaning of process unit; an external automated sampling system connected to sampling port; soft sensors; a signal converter for connection of the sensors to the process control software.

**EXAMPLES**

**Chemicals**

All chemicals and medium components were either purchased from Carl Roth (Karlsruhe, Germany) or Sigma-Aldrich (St. Louis, Missouri, USA).

**Strains and Model Protein**

L-Lactate dehydrogenase 1 (LDH) originating from Lactobacillus plantarum coupled to a his6-tag were expressed in E. coli BL21 (DE3) with an pET28a(+) expression and a T7 promoter.

## Example 1 - *E.coli* Cultivation

E. coli pre-cultures were cultivated in DeLisa defined Medium (DeLisa et al. 1999) supplemented with 50 $\mu$g mL$^{-1}$ kanamycin and 8.8 g L$^{-1}$ glucose. 500 mL of overnight pre-culture were inoculated with 0.5 mL cryo-preserved culture and cultivated (37 °C, 16 h, 250 rpm) in baffled shake flasks in an Infors HR Multitronshaker (Infors AG, Bottmingen, Switzerland). Production of IBs was carried out in a 10 L Techfors-S bioreactor (Infors AG, Bottmingen, Switzerland). The pH was controlled at 7.2 by addition of 2 M phosphoric acid and 12.5 % ammonium hydroxide (v/v). The dissolved oxygen was kept above 40 % by adjustment of stirrer speed (500-900 rpm), oxygen concentration in the gas flow through a mixture of air and pure oxygen (20.95-24.11 % (v/v)) and pressure (0.5-1.0 bar). The batch phase in 4500 mL of DeLisa batch medium (DeLisa et al. 1999), 50 $\mu$g mL$^{-1}$ kanamycin and 22 g L$^{-1}$ glucose was initiated by addition of 500 mL of pre-culture, and was followed by a fed-batch phase with a glucose feed of 440 g L$^{-1}$ and a specific glucose uptake rate ($q_s$) of 0.2 g g$^{-1}$ h$^{-1}$. After 16 h of cultivation, induction was carried out with 1 mM isopropyl-D-thiogalactopyranoside. The cells were harvested after additional 8 h of cultivation and centrifuged (16000 xg, 25 minutes, 4 °C) before the obtained biomass pellet was stored at -20 °C until further processing.

## Example 2 - Homogenization and Inclusion Body Isolation

The thawed biomass was resuspended in lysis buffer (100 mM Tris-HCl, 10 mM Ethylenediaminetetraacetic acid (EDTA), pH 7.4) at a wet weight of 150 g L$^{-1}$. Cell disruption was carried out via three passages of high-pressure homogenization using a PandaPlus 2000 Lab Homogenizer (GEA group, Düsseldorf, Germany) at 1200 bar, followed by centrifugation (13000 xg, 20 minutes, 4 °C), and the discarding of the supernatant. Separation of IBs and cell debris was achieved by a two-step washing process. First, lysed cells were resuspended in washing buffer (50 mM Tris-HCl, 0.5 M NaCl, 0.02 % Tween 80 (w/v), pH 8.0) at a wet weight of 150 g L$^{-1}$ followed by centrifugation (13000 xg, 15 minutes, 4 °C). The supernatant was discarded and the resulting IB pellet was then resuspended in a second washing buffer (50 mM Tris-HCl, 5 mM EDTA, pH 8.0), centrifuged (13000 xg, 15 minutes, 4 °C), and the obtained IB pellet was stored at -20 °C until further usage.

## Example 3 - Solubilization

Solubilization of the IBs was conducted in reaction tubes after thawing of the frozen IBs at room temperature (RT). Solubilization buffer (4 M GuHCl, 150 mM phosphate buffer, pH 6.0) was added to reach a final concentration of 100 g wet IB L$^{-1}$. The mixture was incubated under slight agitation (2 h, RT), centrifuged (20000 xg, 10 minutes, 4 °C), and the supernatant containing the solubilized protein was used for refolding processes.

In the following Examples, if not stated otherwise refolding was carried out in dilution mode in a 1.5 L Labfors 5 stirred tank reactor (Infors AG, Bottmingen, Switzerland) at an initial volume of 0.8 L. The temperature was kept constant at 10 °C by connection of a Lauda Alpha R8 thermostat (Lauda, Königshofen, Germany) to the cooling system of the vessel. Agitation with Rushton turbine impellers was set to 200 rpm, and the pH was controlled at 6.0 by addition of 0.2 M HCl and 0.5 M NaOH. Headspace aeration with pressurized air was kept at a flowrate of 1 vvm.

## Example 4 - Refolding with Process Monitoring

A fed-batch refolding process with constant feeding of solubilized LDH using a preciflow peristaltic pump (Lambda Laboratory Instruments, Brno, Czech Republik) into the refolding vessel containing the refolding buffer (150 mM phosphate buffer, 1 mM EDTA, 20 $\mu$M Nicotinamide adenine dinucleotide, pH 6.0) with and a subsequent batch phase was conducted.

The incoming measurements of native protein and folding intermediates - two protein states of the basic INA model - were used during the update step of the particle-filter for real-time state-estimation (500 particles, systematic resampling). Along the processing time of 26 h samples were taken regularly every 0.5 h during the first 5 h hours, followed by irregular sampling intervals of either 1 h or 2 h, and a lack of measurement update of 16 h.

During the feed phase, a constant increase of native protein was measured while a lower concentration of folding intermediates was present. In this period, the state-estimator accurately predicts the native LDH concentration. While the estimation of the intermediate state deviates during the initial phase of the process, the update step of the particle filter in combination with resampling led to a more accurate prediction of folding intermediates after the third incoming measurement. However, as the folding reaction slowed down after 4 h, indicated by accumulation of intermediate protein and a stagnating increase of native protein, predictions tend to be higher than the actual values in the case of correctly folded LDH and lower than the incoming measurements in the case of the intermediate protein. As real-time measurements of aggregation and total protein content were lacking, both states were solely estimated by the Digital Twin (data not shown). However, data feedback of the two measured states still gave an accurate estimation of the process, highlighting the benefits of using the Digital Twin for monitoring of biological processes.

**Example 5 - Optimizing the Refolding Process -Adjusting the Digital Twin**

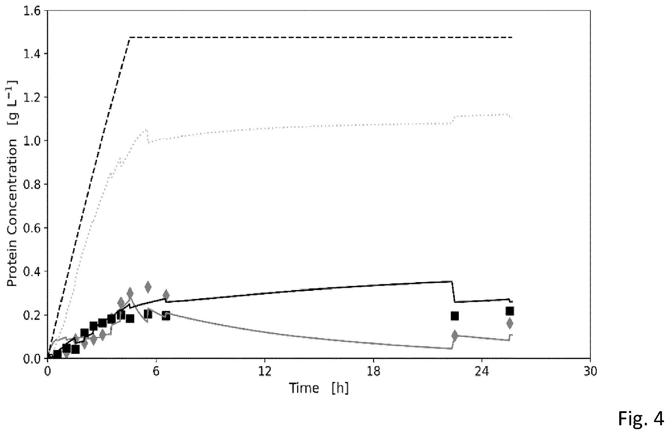

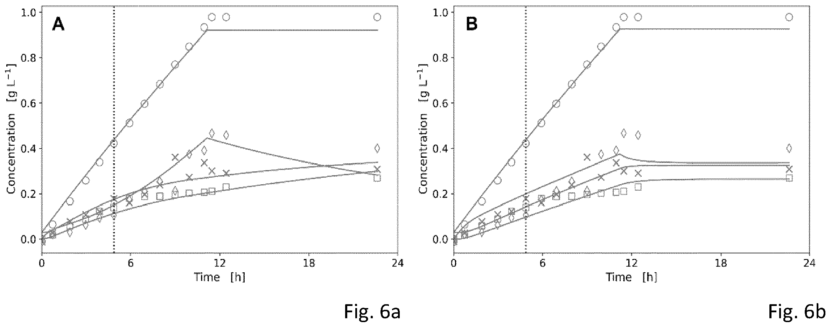

Contrary to the results presented in Figure 4, fed-batch refolding processes are known to be advantageous over batch processes (Pauk et al. 2021). It was shown that a major part of the solubilized protein that was fed into the vessel reacted to aggregates, repressing refolding reactions and thus leading to a final low product recovery of 0.15 g g$^{-1}$ after 26 h of processing time (Figure 5, i). Additionally, the final protein concentration of 1.7 g L$^{-1}$ was higher than usually reported in refolding processes (Eiberle and Jungbauer 2010). To increase productivity by prohibiting aggregation reaction, optimization of the process was conducted by reduction of the final protein concentration on the one hand and reduction of the feed rate on the other hand, assuring beneficial low protein concentrations throughput the process (Figure 5, II). The feed rate of the fed-batch process was calculated based on the basic INA model with the goal of increasing the recovery yield of the process (Figure 5, I and II). Additionally, the final protein concentration of the fed-batch phase was reduced. After feeding, folding was prolonged in a subsequent batch phase with the remaining solubilized protein. Time resolved protein states and model fits are visualized in Figure 6, and corresponding model parameters and state errors can be found in Table 1. Parametrization of the basic model with the presented data was used as basis for the model-based experimental design.

Table 1: Comparison of model parameters and state error of the original and adapted process model for basic fed-batch refolding process with constant feeding (Figure 6).

| Basic INA Model | | | | Adapted INA Model | | | |
|---|---|---|---|---|---|---|---|
| Model parameter | Value | Stat e | NRMS E [%] | Model parameter | Value | Stat e | NRMS E [%] |
| a$_n$ | 0.8200 | I | 10.73 | a$_n$ | 0.5987 | I | 15.03 |
| bn | -12.3520 | N | 8.36 | bn | -0.0499 | N | 11.67 |
| a$_a$ | 30.6243 | A | 18.78 | a$_a$ | 18.6158 | A | 20.71 |
| ba | -22.7334 | D | 0.04 | ba | -0.1719 | D | 0.26 |
| | | V | 0.04 | c$_{I,min}$ | 0.3253 | V | 0.28 |

During the first 5 h of refolding the intermediate state of the protein was mostly converted to native protein or aggregates (Figure 6). As the process progressed, the gain of native LDH decreased and protein in the intermediate conformation accumulated without further reaction potential, making up 40 % of the overall protein concentration at the end of the process. Simultaneously aggregate formation proceeded at a strong pace leading to final aggregation yields of 0.32 g g$^{-1}$ compared to product yields of 0.29 g g$^{-1}$. Fitting the model based on Dong et al. (2004) onto the data results in an accurate simulation of the process. However, the simulation indicates that the concentration of folding intermediates decreases towards zero, but the measurements show that residual folding intermediates remain in the vessel at the end of the process. This effect may be attributed to the accumulating denaturant concentration - possibly destabilizing the native protein conformation (Bernardez Clark et al. 1999) or fixating the target protein in its soluble state (Alibolandi and Mirzahoseini 2011). Furthermore, due to numerous possible folding states arising from the conformational space in high-dimension, the occurrence and accumulation of folding intermediates can be attributed to the protein getting trapped in local energy minima (Clark 2004). Considering this, an extension of the model by a constant term for the residual and non-reacting intermediate state (Figure 5, iv) did not lead to a reduced state error (Table 1), but still led to a more accurate description of the behaviour of the intermediate state and thus the driving force behind the refolding reaction. This model parameter $c_{I,min}$ is scaled by the current total and expected maximal total protein concentration ($c_P$, $c_{P,max}$) and is incorporated into the model only in the reaction part of the differential equations (Equation 1-3) but not into the dilution term, yielding the adapted model of Equations 9-12 together with Equations 4 and 5.

$$c_{I,react} = c_I - c_{Imin} \cdot \frac{c_P}{c_{P,max}} \qquad (10)$$

$$\frac{dc_I}{dt} = -\left(k_n \cdot c_{I,react} + k_a \cdot c_{I,react}^2\right) + \frac{\sum_{k=1}^{r} F_{R,k} \cdot c_{SR,k}}{V} - \frac{\sum_{k=1}^{r} F_{R,k}}{V} \cdot c_I \qquad (11)$$

$$\frac{dc_N}{dt} = k_n \cdot c_{I,react} - \frac{\sum_{k=1}^{r} F_{R,k}}{V} \cdot c_N \tag{12}$$

$$\frac{dc_A}{dt} = k_a \cdot c_{I,react}^2 - \frac{\sum_{k=1}^{r} F_{R,k}}{V} \cdot c_A \tag{13}$$

The underlying reaction rates propose the dependency of refolding and aggregate formation solely based on the concentration of denaturing agent. However, due to the higher order of aggregation dynamics it follows that the overall protein concentration is a critical factor in refolding processes. Indicated by the accumulation of intermediate protein (Figure 5, i) and a lack of state changes shown in Figure 6 (dotted line) it can be assumed that the refolding dynamics are simultaneously influenced by the GuHCl as well as the protein concentration. Thus, to further optimize the process as well as to develop a more suitable model, the interactions between both parameters and the resulting reaction rates of aggregate formation and refolding were further analysed.

**Example 6 -Optimizing the Refolding process - Adjusting CPPs**

6.1 Experimental Preparation

Starting with the parametrized basic model, the focus of analysis lies on the dependency of the reaction kinetics on denaturant and total protein concentration and how to influence the refolding kinetics towards improved yields and lower residual folding Intermediates (Figure 5, i). Decreasing the denaturant concentration in the reservoir of the solubilized feed while maintaining high protein concentrations is a tedious process as additional processing steps are needed to reduce the overall efficiency and reproducibility of the processing chain. Alternatively, both parameters can be controlled by variation of the feed rate and through the introduction of a multi feed system (data not shown, see) leading to the dilution of the concentrations in the refolding vessel by addition of buffer with varying concentrations of denaturing agent (Figure 5, II and ii).

After observing a stagnation of the refolding reaction at around 0.5 g L$^{-1}$ total protein in the fed-batch experiment with reduced feed rate (Figure 6), a two-feed fed-batch process was designed. With the goal of avoiding the accumulation of folding intermediates a second constant feed was started after the critical concentration was reached, assuring concentrations below the critical threshold. The feed rate of this second feed was optimized targeted at diluting the protein concentration in the vessel to a constant level using an evolutionary algorithm in the solver of Microsoft Excel (Fig. 5, II). (Microsoft Corporation, Redmont, USA) (Fig. 8, A).

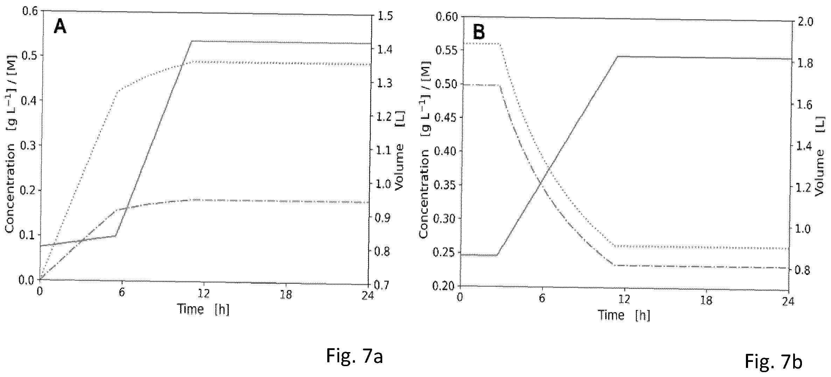

An alternative approach (Figure 7, B) was designed to initiate the refolding process in batch mode at the critical protein concentration of 0.5 g L$^{-1}$, thereby assuming inhibited refolding kinetics. Subsequently after 2.6 h, a constant feed of protein free refolding buffer was used to steadily dilute the remaining solubilized protein (Figure 8 B). This approach was not only targeted at reducing residual folding intermediates while enhancing the refolding dynamics, but also on analyzing the reversibility of reactions as it was shown previously that the intermediate protein confirmation is an energetically favorable state (Figure 5, i), thus being involved in hindering conversion towards the native state of the protein (Clark 2004).

6.2 Refolding Process and Data Evaluation

Comparison of the KPIs (Figure 5, IV) of the initial fed-batch process (Figure 6) to the designed processes based on the proposed model-based experimental design cycle (Figure 5, II) indicate that adjustment of the total protein concentration below the critical threshold led to a two-fold reduction of residual folding intermediates and an increase in product recovery of almost 50 % Figure 7.

**Table 2:** Comparison of KPIs between initial and optimized fed-batch refolding processes with constant feed rates during feed phases.

| Process | Yield [g g$^{-1}$] | Aggregates [g g$^{-1}$] | Residual I [g g$^{-1}$] | Volume Increase [-] |
|---|---|---|---|---|
| Fed-batch | 0.28 | 0.32 | 0.4 | 1.09 |
| Two-feed system | 0.48 | 0.37 | 0.15 | 1.65 |
| Batch dilution | 0.55 | 0.27 | 0.18 | 2.19 |

The process that was initiated in batch phase with subsequent dilution feed showed that accumulation of folding intermediates does not result in irreversible conformational changes. Therefore, it can be assumed that variation of the feed rate in a fed-batch refolding process, e.g., by an MPC, can be used to change ongoing refolding dynamics and thus to influence the KPIs of a process in real-time. Although, the recovery yields of the designed processes show favorable conditions for protein refolding thereby enabling potential for model-based process control of fed-batch systems, the two presented approaches (Figure 7 A and B) do not give insights into the individual influence of denaturant and protein concentration as both parameters were altered simultaneously. Regarding feed control and subsequent influence of the reaction kinetics correlation of the individual process parameters and their weight on the ongoing dynamics is essential. Thus, a third process was designed (Figure 5, II, ii) incorporating three phases, where, after a typical fed-batch phase, additional phases were conducted during which one of the process parameters was kept constant, while the other one was altered (Figure 8, A).

The time resolved differentiation between the individual protein states shown in Figure 8 B indicates that during the first phase folding behavior is similar to observations made previously (Figure 6), where at lower protein concentrations folding intermediates are partially transferred to either the native state or, at a very low rate, are converted to aggregates. After initiation of the second phase, targeted at constant protein and GuHCl levels, due to massive dilution by a second feed, a two-fold increase in the volume was observed, leading to a decline of all protein states including the total protein concentration. However, during the third phase, as expected, the amount of total protein decreased as well as the concentration of folding intermediates, whereas the pace of the dilution of native protein slowed down severely, and aggregates started to occur (Figure 8 B).

Consequently, considering the total amount of states depicted in Figure 8 A, a constant rise of native protein throughout all processing phases can be observed resulting in a high recovery yield of 0.65 g g$^{-1}$. At the same time intermediate accumulation is reduced and maintained at a constant value that is equal to the maximum intermediate state of the first fed-batch phase. Thus, it can be assumed that both parameters, amount of total protein as well as denaturation concentration, have a direct impact on protein folding. However, indicated by a rise in aggregate formation, declining protein concentrations at constant denaturant levels seem to drive the reactions of folding intermediates towards aggregation (Figure 5, IV). This contradicts findings where GuHCl has been used as refolding additive as it was shown that elevated concentrations of denaturing agents yield higher product recovery (Yamaguchi and Miyazaki 2014).

6.3 Model Adaptation and Parametrization

An additional model adaptation was conducted on the reaction rates (Figure 5, iv). By using a three-feed system, separation between the influence of denaturant and total protein concentration was possible. Consequently, the total protein concentration is incorporated in the reaction kinetics in the same way, as the denaturant concentration.

$$k_m = a_m \cdot (1 + c_D)^{b_m} \cdot (1 + c_P)^{c_m} \tag{14}$$

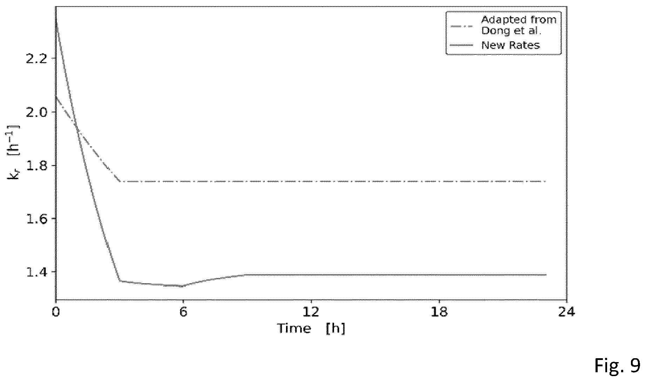

This resulted in a more realistic kinetic (compare Figure 9), where the refolding reaction rate now increases in phase 3 due to decreasing total protein concentration while it declines a lot faster during the initial fed-batch phase than previously assumed. Furthermore, this change resulted in a decreased state error, especially in the aggregates at the cost of two additional model parameters.

Table 3: Comparison of model parameters and state error of the adapted INA model and the further adapted model with the new reaction kinetics depending on both denaturant and total protein concentration for the three-feed system (Figure 8).

| Adapted INA Model | | | | Adapted INA Model with Improved Kinetics | | | |
|---|---|---|---|---|---|---|---|
| Model parameter | Value | State | NRMSE [%] | Model parameter | Value | State | NRMSE [%] |
| $a_n$ | 2.0574 | I | 11.82 | $a_n$ | 2.2706 | I | 11.08 |
| $b_n$ | -1.8661 | N | 8.02 | $b_n$ | -2.1383 | N | 8.08 |
| $a_a$ | 4.1033 | A | 31.76 | $c_n$ | -2.2365 | A | 21.37 |
| $b_a$ | -0.0956 | D | 0.16 | $a_a$ | 7.9766 | D | 0.15 |
| $c_{I,min}$ | 0.0435 | V | 0.11 | $b_a$ | -0.0881 | V | 0.11 |
| | | | | $C_a$ | -0.1434 | | |
| | | | | $c_{I,min}$ | 0.0368 | | |

# EP 4 261 218 A1

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## REFERENCES

Alibolandi, Mona; Mirzahoseini, Hasan (2011): Chemical assistance in refolding of bacterial inclusion bodies. In Biochem Res Int 2011, p. 631607. DOI: 10.1155/2011/631607.

Bade, Pratap D.; Kotu, Susmitha P.; Rathore, Anurag S. (2012): Optimization of a refolding step for a therapeutic fusion protein in the quality by design (QbD) paradigm. In J Sep Sci 35 (22), pp. 3160-3169. DOI: 10.1002/jssc.201200476.

Baeshen, Nabih A.; Baeshen, Mohammed N.; Sheikh, Abdullah; Bora, Roop S.; Ahmed, Mohamed Morsi M.; Ramadan, Hassan A. I. et al. (2014): Cell factories for insulin production. In Microb Cell Fact 13 (1), p. 141. DOI: 10.1186/s12934-014-0141-0.

Bernardez Clark, E. de; Hevehan, D.; Szela, S.; Maachupalli-Reddy, J. (1998): Oxidative renaturation of hen egg-white lysozyme. Folding vs aggregation. In Biotechnol Prog 14 (1), pp. 47-54. DOI: 10.1021/bp970123w.

Bernardez Clark, Eliana de; Schwarz, Elisabeth; Rudolph, Rainer (1999): [15] Inhibition of aggregation side reactions during in vitro protein folding. In : Amyloid, Prions, and Other Protein Aggregates, vol. 309: Elsevier (Methods in Enzymology), pp. 217-236.

BioPharma Market: An Inside Look (2018). Available online at https://pharmaceutical.report/resources/whitepapers/f879ef54-b868-445a-9e9f-1f03821a0685_pm1902-biomanufacturing-trends.pdf#page=4.

Chen, Ying; Wang, Qi; Zhang, Chun; Li, Xiunan; Gao, Qiang; Dong, Changqing et al. (2016): Improving the refolding efficiency for proinsulin aspart inclusion body with optimized buffer compositions. In Protein Expr Purif 122, pp. 1-7. DOI: 10.1016/j.pep.2016.01.015.

Clark, Patricia L. (2004): Protein folding in the cell: reshaping the folding funnel. In Trends Biochem Sci 29 (10), pp. 527-534. DOI: 10.1016/j.tibs.2004.08.008.

Daume, Sven; Kofler, Sandro; Kager, Julian; Kroll, Paul; Herwig, Christoph (2020): Generic Workflow for the Setup of Mechanistic Process Models. In : Animal Cell Biotechnology: Humana, New York, NY, pp. 189-211.

DeLisa, Matthew P.; Li, Jincai; Rao, Govind; Weigand, William A.; Bentley, William E. (1999): Monitoring GFP-operon fusion protein expression during high cell density cultivation of Escherichia coli using an on-line optical sensor. In Biotechnol Bioeng 65 (1), pp. 54-64. DOI: 10.1002/(SICI)1097-0290(19991005)65:1<54::AID-BIT7>3.0.CO;2-R.

Dong, Xiao-Yan; Shi, Guang-Quan; Li, Wei; Sun, Yan (2004): Modeling and simulation of fed-batch protein refolding process. In Biotechnol Prog 20 (4), pp. 1213-1219. DOI: 10.1021/bp0499597.

Eiberle, Maria K.; Jungbauer, Alois (2010): Technical refolding of proteins: Do we have freedom to operate? In Biotechnol J 5 (6), pp. 547-559. DOI: 10.1002/biot.201000001.

FDA (2004): PAT - a framework for innovative pharmaceutical development, manufacturing, and quality assurance. U.S. Department of Health and Human Services, Food and Drug Administration.

Fifteen years of progress: biopharmaceutical industry survey results (2018). Available online at http://alfresco-static-files.s3.amazonaws.com/alfresco_images/pharma/2018/10/11/0592de88-9026-4c61-b660-f5fd790b7cea/pharmtech_europe_july2018.pdf#page=10.

García-Fruitós, Elena (2010): Inclusion bodies: a new concept. In Microb Cell Fact 9 (1), p. 80. DOI: 10.1186/1475-2859-9-80.

Gerzon, Gabriella; Sheng, Yi; Kirkitadze, Marina (2022): Process Analytical Technologies - Advances in bioprocess integration and future perspectives. In J Pharm Biomed 207, p. 114379. DOI: 10.1016/j.jpba.2021.114379.

Guin, Drishti; Sye, Kori; Dave, Kapil; Gruebele, Martin (2016): Dodine as a transparent protein denaturant for circular dichroism and infrared studies. In Protein Sci 25 (5), pp. 1061-1068. DOI: 10.1002/pro.2914.

Humer, Diana; Ebner, Julian; Spadiut, Oliver (2020): Scalable High-Performance Production of Recombinant Horseradish Peroxidase from E. coli Inclusion Bodies. In Int J Mol Sci 21 (13). DOI: 10.3390/ijms21134625.

Humer, Diana; Spadiut, Oliver (2018): Wanted: more monitoring and control during inclusion body processing. In World J Microbiol Biotechnol 34 (11), p. 158. DOI: 10.1007/s11274-018-2541-5.

Kesik-Brodacka, Malgorzata (2018): Progress in biopharmaceutical development. In Biotechnol Appl Biochem 65 (3), pp. 306-322. DOI: 10.1002/bab.1617.

Overton, Tim W. (2014): Recombinant protein production in bacterial hosts. In Drug Discov Today 19 (5), pp. 590-601. DOI: 10.1016/j.drudis.2013.11.008.

Pan, Siqi; Odabas, Nora; Sissolak, Bernhard; Imendorffer, Moritz; Zelger, Monika; Jungbauer, Alois; Hahn, Rainer (2015): Engineering batch and pulse refolding with transition of aggregation kinetics: An investigation using green fluorescent protein (GFP). In Chem Eng Sci 131, pp. 91-100. DOI: 10.1016/j.ces.2015.03.054.

Pathak, Mili; Dixit, Shruti; Muthukumar, S.; Rathore, Anurag S. (2016): Analytical characterization of in vitro refolding in the quality by design paradigm: Refolding of recombinant human granulocyte colony stimulating factor. In J Pharm Biomed 126, pp. 124-131. DOI: 10.1016/j.jpba.2016.05.001.

Pauk, Jan Niklas; Raju Palanisamy, Janani; Kager, Julian; Koczka, Krisztina; Berghammer, Gerald; Herwig, Christoph; Veiter, Lukas (2021): Advances in monitoring and control of refolding kinetics combining PAT and modeling. In Appl Microbiol Biotechnol 105 (6), pp. 2243-2260. DOI: 10.1007/s00253-021-11151-y.

Pham, Janette V.; Yilma, Mariamawit A.; Feliz, Adriana; Majid, Murtadha T.; Maffetone, Nicholas; Walker, Jorge R. et al. (2019): A Review of the Microbial Production of Bioactive Natural Products and Biologics. In Front Microbiol 10, p. 1404. DOI: 10.3389/fmicb.2019.01404.

Rathore, Anurag S. (2014): QbD/PAT for bioprocessing: moving from theory to implementation. In Curr Opin in Chem Eng6, pp. 1-8. DOI: 10.1016/j.coche.2014.05.006.

Sanchez-Garcia, Laura; Martin, Lucas; Mangues, Ramon; Ferrer-Miralles, Neus; Vázquez, Esther; Villaverde, Antonio (2016): Recombinant pharmaceuticals from microbial cells: a 2015 update. In Microb Cell Fact 15 (1), p. 33. DOI: 10.1186/s12934-016-0437-3.

Schlegl, Robert; Tscheliessnig, Anne; Necina, Roman; Wandl, Robert; Jungbauer, Alois (2005): Refolding of proteins in a CSTR. In Chem Eng Sci 60 (21), pp. 5770-5780. DOI: 10.1016/j.ces.2005.04.086.

Singh, Surinder Mohan; Panda, Amulya Kumar (2005): Solubilization and refolding of bacterial inclusion body proteins. In J Biosci Bioeng 99 (4), pp. 303-310. DOI: 10.1263/jbb.99.303.

Singhvi, Priyank; Saneja, Ankit; Srichandan, Sudeepa; Panda, Amulya K. (2020): Bacterial Inclusion Bodies: A Treasure Trove of Bioactive Proteins. In Trends Biotechnol 38 (5), pp. 474-486. DOI: 10.1016/j.tibtech.2019.12.011.

Villaverde, Alejandro F.; Tsiantis, Nikolaos; Banga, Julio R. (2019): Full observability and estimation of unknown inputs, states and parameters of nonlinear biological models. In J R Soc Interface 16 (156), p. 20190043. DOI: 10.1098/rsif.2019.0043.

Walther, Cornelia; Mayer, Sabrina; Jungbauer, Alois; Dürauer, Astrid (2014): Getting ready for PAT: Scale up and inline monitoring of protein refolding of Npro fusion proteins. In

Yamaguchi, Hiroshi; Miyazaki, Masaya (2014): Refolding techniques for recovering biologically active recombinant proteins from inclusion bodies. In Biomolecules 4 (1), pp. 235-251. DOI: 10.3390/biom4010235.

**Claims**

1.  A method for optimizing the production process of a protein, comprising a refolding process, using a Digital Twin of the production process, comprising the following steps:

    • generating the Digital Twin of the production process by reproducing the refolding process and optionally further unit operations by means of mathematical models and known values of critical process parameters (CPPs), and process determinants selected from a critical quality attributes (CQAs) and key performance indicators (KPIs) of the unit operation,
    • varying the input of at least one CPP within a predetermined range in the Digital Twin and calculating predicted values or the development over time of at least one process determinant in the Digital Twin;
    • determining the input of the CPP for which an optimal value or development over time of the process determinant is predicted in the Digital Twin as the optimally predicted CPP input; and
    • adjusting the CPP input to the optimal predicted CPP input when performing the production process.

2.  The method of claim 1, wherein the method comprises the further steps of:

    • measuring the value or development over time of the process determinant and,
    • determining the deviation of the measured value from the predicted value of the process determinant or the deviation of the measured development over time from the predicted development over time of the process determinant.

3.  The method of claim 2, wherein if the deviation is greater than a threshold value, the method comprises the further steps of:

    • feeding the pair of values of the CPP and process determinant into the Digital Twin; and
    • updating the mathematical models in the Digital Twin.

4.  The method of claim 2, wherein, if the deviation is less than a threshold value, the method comprises the further steps of:

    ◦ varying the values of at least one second CPP within a predetermined range in the Digital Twin, and calculating the predicted value or predicted development over time of the process determinant in the Digital Twin;
    ◦ determining the value of the second CPP for which an optimal predicted value of the process determinant is calculated in the Digital Twin as the optimally predicted second CPP value; and
    ◦ setting the second CPP to the optimally predicted second CPP value when performing the unit operation of the manufacturing process.

5.  A process according to any one of the preceding claims, wherein

    • the CPPs for the refolding process are selected from: type of buffer, buffer concentration, ionic strength, pH, temperature, pressure, mechanical energy input, starting concentration of protein, mode of addition of the protein, protein feed rate, mode of addition of the refolding buffer, buffer feed rate, protein addition time, redox potential and redox partners, type of co-factors (additives), concentration of co-factors, type of refolding technique, type of denaturant, feed rate of the denaturing agent, denaturing agent addition time, concentration of denaturant , duration of the refolding process.

6.  The method of any one of the preceding claims, wherein the unit operation is selected from the solubilization process, and an inclusion body recovery step selected from cell cultivation, isolation of the inclusion bodies, and washing of the inclusion bodies.

7. A method for producing a protein comprising a refolding process, wherein the refolding process is carried out in a bioreactor and is monitored and/or controlled by means of a Digital Twin of the refolding process, wherein the Digital Twin comprises a state-observer algorithm in combination with a model of the refolding process based mathematical models of the reaction kinetics and known values of CPPs and process determinants selected from CQAs and KPIs of the refolding process, comprising the steps of:

   • feeding the input of CPPs and process determinants selected for the refolding process into the Digital Twin and initializing the state-observer algorithm, wherein the initializing comprises defining a set of process determinants and optionally CPPs, as the initial condition of the process; and
   • starting the refolding process in the bioreactor with the selected input of the CPPs and process determinants.

8. The method of claim 7, comprising the step of measuring one or more of the process determinants of the defined set in the bioreactor.

9. The method of claim 8, wherein after the lapse of a monitoring time step, the Digital Twin performs the step of predicting the temporal evolution of the set of process determinants and optionally CPPs defining the process condition during the monitoring time step based on the model of the refolding process, wherein preferably the step of predicting is iteratively repeated after the lapse of each monitoring time step, wherein preferably the monitoring time step is in the range of 30 sec to 60 min, more preferably the monitoring time step is in the range of 1 min to 20 min, most preferably the monitoring time step is in the range of 2 min to 5 min.

10. The method of claim 9, wherein the state observer is a particle filter algorithm.

11. The method of any of claims 7 to 10, wherein the Digital Twin controls the refolding process and comprises a with a model predictive controller, the method further comprises the steps of:

   • calculating in the Digital Twin the predicted temporal evolution of at least one process determinant for a current and alternative inputs of one or more CPPs, wherein the calculation is based on the process determinant value predicted at the start of the prediction horizon and optionally the measured value of the at least one process determinant;
   • determining which of the temporal evolutions of the measured process determinant minimizes the cost function based on the set point of the at least one process determinant, wherein this is defined as the optimal temporal evolution; and
   • setting the one or more CPPs to the input associated with the optimal temporal evolution;

   wherein the prediction horizon is at least two-fold the monitoring time step, preferably the prediction horizon is in the range of 3 times to 25 times the monitoring time step, more preferably in the range of 5 times to 15 times the monitoring time step.

12. The method according to any one of claims 7 to 11, wherein the CPPs are selected from: pH, temperature, pressure, mechanical energy input, volume of the refolding solution, protein feed rate, protein addition time, concentration of redox buffers, feed rate of the denaturing agent, denaturing agent addition time and concentration of the denaturing agent.

13. The method of any one of claims 7 to 12, wherein the predicted process determinant is selected from the concentration of solubilized protein, the concentration of intermediate state, the concentration of aggregated protein, and the concentration of native protein.

14. The method of any one of the previous claims, wherein the protein

   • has an amino acid chain length of at most 1000 amino acids, preferably at most 700 amino acids, more preferably at most 500 amino acids, most preferably at most 350 amino acids;
   • has at most 10 disulfide bridges, preferably at most 4 disulfide bridges, more preferably 2 disulfide bridges, most preferably no disulfide bridge; and/or
   • has at most 6 subunits, preferably at most 4 subunits, more preferably at most 2 subunits.

15. The method according to claim 14, wherein the protein is selected from lactate dehydrogenase (LDH), galactose oxidase (GalOx), human insulin, interferon $\alpha$2a, filgrastim (G-CSF analog), interferon $\alpha$2a, glucagon, long-acting

insulin glargine, human parathyroid hormone and human granulocyte stimulating factor (GCSF).

16. The method according to any one of the previous claims, the underlying mathematical model of the reaction kinetics of the refolding process is a four-state model, with the states solubilized protein (S), intermediate state (I), aggregated protein (A), and native protein (N), with a rapid reaction from S to I determined by the reaction rate $k_i$, a first-order reaction from I to N and determined by the reaction rate $k_n$, and a higher-order reaction from I to A determined by the reaction rate $k_a$.

17. A bioreactor for the refolding process of proteins, comprising a process vessel, a temperature control, a gassing unit for the addition of one or more of $N_2$, $O_2$ and process air, at least 5 addition groups, one or more sensors and an aseptic sampling port connected to an on-line or at-line measurement unit for one or more process determinants selected from the concentration of solubilized protein, the concentration of intermediate state, the concentration of aggregated protein and the concentration of native protein.

18. A bioreactor according to claim 17, wherein the addition groups are for the addition of the solubilized protein in refolding buffer, an acid, a base, a redox buffer, and the refolding buffer and denaturing agent in refolding buffer, wherein the bioreactor comprises preferably at least 6 addition groups, more preferably at least 7 addition groups.

19. A bioreactor system comprising

   • a bioreactor according to claim 17 or 18;
   • a control system with a process control software, wherein the control system is connected to and control the temperature control, a gassing unit for the addition of one or more of $N_2$, $O_2$ and process air, at least five addition groups;
   • a signal converter connected to the sensors of the bioreactor; and
   • a Digital Twin of the refolding process, wherein the Digital Twin was generated by mapping the refolding process by means of mathematical models of the reaction kinetics and known values of the CPPs and process determinants selected from CQAs and KPIs of the refolding process, implemented on a computer, wherein the Digital Twin is connected to the control system and the signal converter.

Fig. 1

Fig. 2

**Refolding Process**

**Digital Twin**

Data Evaluation

Process Control Software

User Input

Data Input

State-Observer Algorithm

Model-Predictive Control Algorithm

Data Output

Fig. 3

EP 4 261 218 A1

Fig. 4

Fig. 5

Fig. 6b

Fig. 6a

Fig. 7b

Fig. 7a

Fig. 8a

Fig. 8b

Fig. 8c

Fig. 9

Fig. 10

Fig. 11

**EUROPEAN SEARCH REPORT**

Application Number

EP 22 16 7691

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | PAUK JAN NIKLAS ET AL: "Advances in monitoring and control of refolding kinetics combining PAT and modeling", APPLIED MICROBIOLOGY AND BIOTECHNOLOGY, vol. 105, no. 6, 17 February 2021 (2021-02-17), pages 2243-2260, XP037412072, ISSN: 0175-7598, DOI: 10.1007/S00253-021-11151-Y * Abstract; figures 2, 3, sections "Monitoring of refolding processes", "Control of refolding processes" and "Proposal for a model-based refolding control strategy; page 2245, left column, top paragraph; page 2251; page 2254, left column, lowest paragraph * ----- | 1-19 | INV. C07K1/00 C07K1/113 G05B13/00 G06N3/00 G16B15/20 |
| A | HELGERS HERIBERT ET AL: "Towards Autonomous Operation by Advanced Process Control-Process Analytical Technology for Continuous Biologics Antibody Manufacturing", PROCESSES, vol. 9, no. 1, 18 January 2021 (2021-01-18), page 172, XP055964446, DOI: 10.3390/pr9010172 Retrieved from the Internet: URL:https://pdfs.semanticscholar.org/4529/8d5c1d992b64a313fd3964e63cd299045cd3.pdf?_ga=2.189466731.1581999742.1663854401-1853568517.1657273118> * the whole document * ----- -/-- | 1-19 | TECHNICAL FIELDS SEARCHED (IPC) C07K |

The present search report has been drawn up for all claims

| Place of search | Date of completion of the search | Examiner |
|---|---|---|
| The Hague | 25 September 2022 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

Europäisches
Patentamt

European
Patent Office

Office européen
des brevets

# EUROPEAN SEARCH REPORT

**Application Number**

EP 22 16 7691

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | OLE LAUSTSEN LAVE ET AL: "Towards a Digital Twin: A validated Digital Model of a Pilot Scale Bioreactor producing Green Fluorescent Protein (GFP)", COMPUTER - AIDED CHEMICAL ENGINEERING, ELSEVIER, NL, vol. 50, no. Pt. 1, 25 June 2021 (2021-06-25), pages 625-630, XP009539351, ISSN: 1570-7946, DOI: 10.1016/B978-0-323-88506-5.50099-1 [retrieved on 2021-07-18] * the whole document * | 1-19 | |
| A | LOHMANN LARA JULIA ET AL: "Process Analytical Technology for Precipitation Process Integration into Biologics Manufacturing towards Autonomous Operation-mAb Case Study", PROCESSES, vol. 9, no. 3, 9 March 2021 (2021-03-09), page 488, XP055964447, DOI: 10.3390/pr9030488 * the whole document * | 1-19 | |
| A | VISHWANATH HEBBI ET AL: "Process analytical technology implementation for protein refolding: GCSF as a case study", BIOTECHNOLOGY AND BIOENGINEERING, JOHN WILEY, HOBOKEN, USA, vol. 116, no. 5, 18 January 2019 (2019-01-18), pages 1039-1052, XP071166068, ISSN: 0006-3592, DOI: 10.1002/BIT.26900 * the whole document * | 1-19 | TECHNICAL FIELDS SEARCHED (IPC) |

The present search report has been drawn up for all claims

| Place of search | Date of completion of the search | Examiner |
|---|---|---|
| The Hague | 25 September 2022 | Burema, Shiri |

EPO FORM 1503 03.82 (P04C01)

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALIBOLANDI, MONA ; MIRZAHOSEINI, HASAN.** Chemical assistance in refolding of bacterial inclusion bodies. *Biochem Res Int,* 2011, vol. 2011, 631607
- **BADE, PRATAP D. ; KOTU, SUSMITHA P. ; RATHORE, ANURAG S.** Optimization of a refolding step for a therapeutic fusion protein in the quality by design (QbD) paradigm. *J Sep Sci,* 2012, vol. 35 (22), 3160-3169
- **BAESHEN, NABIH A. ; BAESHEN, MOHAMMED N. ; SHEIKH, ABDULLAH ; BORA, ROOP S. ; AHMED, MOHAMED MORSI M. ; RAMADAN, HASSAN A. I. et al.** Cell factories for insulin production. *Microb Cell Fact,* 2014, vol. 13 (1), 141
- **BERNARDEZ CLARK, E. DE ; HEVEHAN, D. ; SZELA, S. ; MAACHUPALLI-REDDY, J.** Oxidative renaturation of hen egg-white lysozyme. Folding vs aggregation. *Biotechnol Prog,* 1998, vol. 14 (1), 47-54
- Inhibition of aggregation side reactions during in vitro protein folding. In : Amyloid, Prions, and Other Protein Aggregates. **BERNARDEZ CLARK, ELIANA DE ; SCHWARZ, ELISABETH ; RUDOLPH, RAINER.** Methods in Enzymology. Elsevier, 1999, vol. 309, 217-236
- *BioPharma Market: An Inside Look,* 2018, https://pharmaceutical.report/resources/whitepapers/f879ef54-b868-445a-9e9f-1f03821a0685_pm1902-biomanufacturing-trends.pdf#page=4
- **CHEN, YING ; WANG, QI ; ZHANG, CHUN ; LI, XIUNAN ; GAO, QIANG ; DONG, CHANGQING et al.** Improving the refolding efficiency for proinsulin aspart inclusion body with optimized buffer compositions. *Protein Expr Purif,* 2016, vol. 122, 1-7
- **CLARK, PATRICIA L.** Protein folding in the cell: reshaping the folding funnel. *Trends Biochem Sci,* 2004, vol. 29 (10), 527-534
- **DAUME, SVEN ; KOFLER, SANDRO ; KAGER, JULIAN ; KROLL, PAUL ; HERWIG, CHRISTOPH.** Generic Workflow for the Setup of Mechanistic Process Models. *Animal Cell Biotechnology,* 2020, 189-211
- **DELISA, MATTHEW P. ; LI, JINCAI ; RAO, GOVIND ; WEIGAND, WILLIAM A. ; BENTLEY, WILLIAM E.** Monitoring GFP-operon fusion protein expression during high cell density cultivation of Escherichia coli using an on-line optical sensor. *Biotechnol Bioeng,* 1999, vol. 65 (1), 54-64
- **DONG, XIAO-YAN ; SHI, GUANG-QUAN ; LI, WEI ; SUN, YAN.** Modeling and simulation of fed-batch protein refolding process. *Biotechnol Prog,* 2004, vol. 20 (4), 1213-1219
- **EIBERLE, MARIA K. ; JUNGBAUER, ALOIS.** Technical refolding of proteins: Do we have freedom to operate?. *Biotechnol J,* 2010, vol. 5 (6), 547-559
- PAT - a framework for innovative pharmaceutical development, manufacturing, and quality assurance. U.S. Department of Health and Human Services, Food and Drug Administration, 2004
- *Fifteen years of progress: biopharmaceutical industry survey results,* 2018, http://alfresco-static-files.s3.amazonaws.com/alfresco_images/pharma/2018/10/11/0592de88-9026-4c61-b660-f5fd790b7cea/pharmtech_europe_july2018.pdf#page=10
- **GARCÍA-FRUITÓS, ELENA.** Inclusion bodies: a new concept. *Microb Cell Fact,* 2010, vol. 9 (1), 80
- **GERZON, GABRIELLA ; SHENG, YI ; KIRKITADZE, MARINA.** Process Analytical Technologies - Advances in bioprocess integration and future perspectives. *J Pharm Biomed,* 2022, vol. 207, 114379
- **GUIN, DRISHTI ; SYE, KORI ; DAVE, KAPIL ; GRUEBELE, MARTIN.** Dodine as a transparent protein denaturant for circular dichroism and infrared studies. *Protein Sci,* 2016, vol. 25 (5), 1061-1068
- **HUMER, DIANA ; EBNER, JULIAN ; SPADIUT, OLIVER.** Scalable High-Performance Production of Recombinant Horseradish Peroxidase from E. coli Inclusion Bodies. *Int J Mol Sci,* 2020, vol. 21 (13
- **HUMER, DIANA ; SPADIUT, OLIVER.** Wanted: more monitoring and control during inclusion body processing. *World J Microbiol Biotechnol,* 2018, vol. 34 (11), 158
- **KESIK-BRODACKA, MALGORZATA.** Progress in biopharmaceutical development. *Biotechnol Appl Biochem,* 2018, vol. 65 (3), 306-322
- **OVERTON, TIM W.** Recombinant protein production in bacterial hosts. *Drug Discov Today,* 2014, vol. 19 (5), 590-601

- **PAN, SIQI ; ODABAS, NORA ; SISSOLAK, BERNHARD ; IMENDORFFER, MORITZ ; ZELGER, MONIKA ; JUNGBAUER, ALOIS ; HAHN, RAINER.** Engineering batch and pulse refolding with transition of aggregation kinetics: An investigation using green fluorescent protein (GFP). *Chem Eng Sci,* 2015, vol. 131, 91-100
- **PATHAK, MILI ; DIXIT, SHRUTI ; MUTHUKUMAR, S. ; RATHORE, ANURAG S.** Analytical characterization of in vitro refolding in the quality by design paradigm: Refolding of recombinant human granulocyte colony stimulating factor. *J Pharm Biomed,* 2016, vol. 126, 124-131
- **PAUK, JAN NIKLAS ; RAJU PALANISAMY, JANANI ; KAGER, JULIAN ; KOCZKA, KRISZTINA ; BERGHAMMER, GERALD ; HERWIG, CHRISTOPH ; VEITER, LUKAS.** Advances in monitoring and control of refolding kinetics combining PAT and modeling. *Appl Microbiol Biotechnol,* 2021, vol. 105 (6), 2243-2260
- **PHAM, JANETTE V. ; YILMA, MARIAMAWIT A. ; FELIZ, ADRIANA ; MAJID, MURTADHA T. ; MAFFETONE, NICHOLAS ; WALKER, JORGE R. et al.** A Review of the Microbial Production of Bioactive Natural Products and Biologics. *Front Microbiol,* 2019, vol. 10, 1404
- **RATHORE, ANURAG S.** QbD/PAT for bioprocessing: moving from theory to implementation. *Curr Opin in Chem Eng,* 2014, vol. 6, 1-8
- **SANCHEZ-GARCIA, LAURA ; MARTIN, LUCAS ; MANGUES, RAMON ; FERRER-MIRALLES, NEUS ; VÁZQUEZ, ESTHER ; VILLAVERDE, ANTONIO.** Recombinant pharmaceuticals from microbial cells: a 2015 update. *Microb Cell Fact,* 2016, vol. 15 (1), 33
- **SCHLEGL, ROBERT ; TSCHELIESSNIG, ANNE ; NECINA, ROMAN ; WANDL, ROBERT ; JUNGBAUER, ALOIS.** Refolding of proteins in a CSTR. *Chem Eng Sci,* 2005, vol. 60 (21), 5770-5780
- **SINGH, SURINDER MOHAN ; PANDA, AMULYA KUMAR.** Solubilization and refolding of bacterial inclusion body proteins. *J Biosci Bioeng,* 2005, vol. 99 (4), 303-310
- **SINGHVI, PRIYANK ; SANEJA, ANKIT ; SRICHANDAN, SUDEEPA ; PANDA, AMULYA K.** Bacterial Inclusion Bodies: A Treasure Trove of Bioactive Proteins. *Trends Biotechnol,* 2020, vol. 38 (5), 474-486
- **VILLAVERDE, ALEJANDRO F. ; TSIANTIS, NIKOLAOS ; BANGA, JULIO R.** Full observability and estimation of unknown inputs, states and parameters of nonlinear biological models. *J R Soc Interface,* 2019, vol. 16 (156), 20190043
- **WALTHER, CORNELIA ; MAYER, SABRINA ; JUNGBAUER, ALOIS ; DÜRAUER, ASTRID.** *Getting ready for PAT: Scale up and inline monitoring of protein refolding of Npro fusion proteins,* 2014
- **YAMAGUCHI, HIROSHI ; MIYAZAKI, MASAYA.** Refolding techniques for recovering biologically active recombinant proteins from inclusion bodies. *Biomolecules,* 2014, vol. 4 (1), 235-251